# EUROPEAN PATENT SPECIFICATION

(11) **EP 4 122 483 B1**
(45) Date of publication and mention of the grant of the patent: **30.04.2025**
(21) Application number: 21772438.4
(22) Date of filing: 17.03.2021
(51) Int. Cl.: A61K 9/00, A61K 38/07, A61K 9/20, A61K 9/48, A61K 47/20, A61P 25/04, A61K 47/18, A61K 9/08, A61K 31/438

(54) **ORAL PHARMACEUTICAL COMPOSITION**
ORALE PHARMAZEUTISCHE ZUSAMMENSETZUNG
COMPOSITION PHARMACEUTIQUE ORALE

(30) Priority: 18.03.2020 CN 202010179149; 12.03.2021 CN 202110268405
(43) Date of publication of application: 25.01.2023
(73) Proprietor: Xizang Haisco Pharmaceutical Co., Ltd., Lhoka, Tibet 856099 (CN)
(72) Inventor: FU, Ling, Chengdu, Sichuan 611130 (CN); WANG, Shunhong, Chengdu, Sichuan 611130 (CN); REN, Dong, Chengdu, Sichuan 611130 (CN); LI, Xiaoping, Chengdu, Sichuan 611130 (CN); MO, Yi, Chengdu, Sichuan 611130 (CN)
(74) Representative: Casalonga
(86) International application number: PCT/CN2021/081224
(87) International publication number: WO 2021/185265

(56) References cited:
- WO-A1-2010/020978
- WO-A1-2010/020978
- WO-A1-2019/015644
- WO-A1-2019/015644
- WO-A1-2019/015644
- WO-A1-2019/042247
- WO-A1-2019/042247
- CN-A- 101 574 343
- CN-A- 101 574 343
- CN-A- 101 627 049
- CN-A- 101 627 049
- CN-A- 108 135 962
- CN-A- 108 135 962
- CN-A- 109 862 906
- CN-A- 109 862 906
- US-A1- 2002 094 346
- US-A1- 2002 094 346
- US-A1- 2009 263 479
- US-A1- 2009 263 479
- MORTEN ASSER KARSDAL ET AL: "Lessons learned from the clinical development of oral peptides", BRITISH JOURNAL OF CLINICAL PHARMACOLOGY, BLACKWELL SCIENTIFIC PUBL, GB, vol. 79, no. 5, 22 April 2015 (2015-04-22), pages 720 - 732, XP071601829, ISSN: 0306-5251, DOI: 10.1111/BCP.12557
- CAROLINE TWAROG ET AL: "Intestinal Permeation Enhancers for Oral Delivery of Macromolecules: A Comparison between Salcaprozate Sodium (SNAC) and Sodium Caprate (C10)", PHARMACEUTICS, vol. 11, no. 2, 13 February 2019 (2019-02-13), pages 78, XP055634457, DOI: 10.3390/pharmaceutics11020078
- DOI NOBUYUKI ET AL: "Absorption Enhancement Effect of Acylcarnitines through Changes in Tight Junction Protein in Caco-2 Cell Monolayers", DRUG METABOLISM AND PHARMACOKINETICS, vol. 26, no. 2, 1 January 2011 (2011-01-01), JP, pages 162 - 170, XP93129477, ISSN: 1347-4367, DOI: 10.2133/dmpk.DMPK-10-RG-071
- CAROLINE TWAROG ET AL: "Intestinal Permeation Enhancers for Oral Delivery of Macromolecules: A Comparison between Salcaprozate Sodium (SNAC) and Sodium Caprate (C10)", PHARMACEUTICS, vol. 11, no. 2, 13 February 2019 (2019-02-13), pages 78, XP055634457, DOI: 10.3390/pharmaceutics11020078
- MORTEN ASSER KARSDAL ET AL: "Lessons learned from the clinical development of oral peptides", BRITISH JOURNAL OF CLINICAL PHARMACOLOGY, BLACKWELL SCIENTIFIC PUBL, GB, vol. 79, no. 5, 22 April 2015 (2015-04-22), pages 720 - 732, XP071601829, ISSN: 0306-5251, DOI: 10.1111/BCP.12557
- DOI NOBUYUKI ET AL: "Absorption Enhancement Effect of Acylcarnitines through Changes in Tight Junction Protein in Caco-2 Cell Monolayers", DRUG METABOLISM AND PHARMACOKINETICS, vol. 26, no. 2, 1 January 2011 (2011-01-01), JP, pages 162 - 170, XP093129477, ISSN: 1347-4367, DOI: 10.2133/dmpk.DMPK-10-RG-071

## Description

### Technical Field

The present invention relates to an oral pharmaceutical composition of a peptide amide compound. The present invention further relates to a method for preparing the oral pharmaceutical composition and the use thereof.

### Background Art

Polypeptide drugs can be used for treating various diseases due to its wide indications, high safety, remarkable selectivity and effectiveness. With the development of biotechnology, a large number of peptide drugs have entered the market. Currently, the commercially available peptide preparations are mainly in the form of injections and nasal sprays, which bring great inconvenience to patients. For patients, the oral administration is simpler and does not directly damage the skin or mucous membranes, thereby reducing patients' pain and improving patient compliance. However, the oral administration of polypeptide drugs is limited by a series of barriers, specifically, the polypeptide drugs are easily degraded and destroyed by proteolytic enzymes in the digestive tract, and are difficult to pass through the epithelial cell layer of the inner wall of the small intestine due to the large molecular weight, resulting in low oral bioavailability and limiting the clinical use thereof. For example, the absolute bioavailability of the listed semaglutide tablets is only 0.4%-1%. In order to achieve the blood concentration required for the onset of action, the oral dose is relatively high, and it is difficult to achieve oral administration of some polypeptide drugs with a high cost or a narrow therapeutic window. Therefore, improving the oral bioavailability of polypeptide drugs is the key to achieving the oral administration of polypeptide drugs.

Most polypeptide drugs are susceptible to degradation by a protease activated by gastric acid, resulting in a decrease in the activity thereof. Enteric coating technology is usually used to protect polypeptides, which, however, requires complicated process and is of high cost.

WO 2019/015644 discloses a class of peptide amide compounds with novel structure and good analgesic effect, and the general formula thereof is as follows: 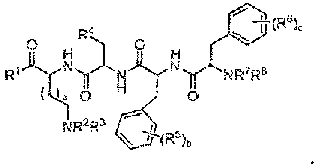 Such compounds have a significant agonizing effect on human κ-opioid receptors. Specifically, compound A is disclosed therein: 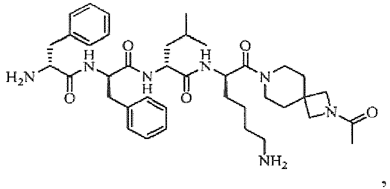 with a chemical name of 7-(D-phenylalanyl-D-phenylalanyl-D-leucyl-D-lysyl)-2-acetyl-2,7-diazaspiro[3.5]nonane.

### Summary of the Invention

The present invention provides an oral polypeptide pharmaceutical composition, which is prepared by combining a polypeptide drug with a specific absorption enhancer and other non-active ingredients, thereby promoting the permeability of the polypeptide drug in the gastrointestinal tract, improving the oral bioavailability of the polypeptide drug, and achieving the oral administration so as to improve patient compliance.

The present invention provides a simple intragastric administration technology, which greatly simplifies the technological process and can greatly improve the oral bioavailability of the polypeptide compound A.

One object of the present invention is to provide an oral pharmaceutical composition of compound A:

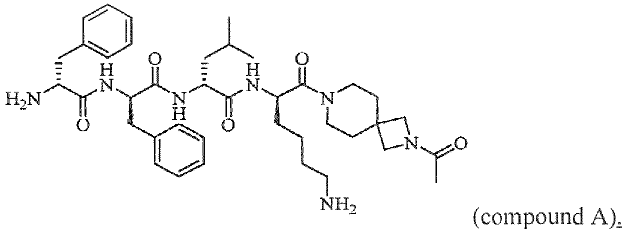

### and of an absorption enhancer as defined below.

The present invention significantly improves the oral bioavailability of compound A, and is more favorable to develop a drug with the compound A.

Another object of the present invention is to provide a method for preparing the oral pharmaceutical composition.

Another object of the present invention is to provide the use of the oral pharmaceutical composition.

The present invention provides an oral pharmaceutical composition comprising:
a) compound A: 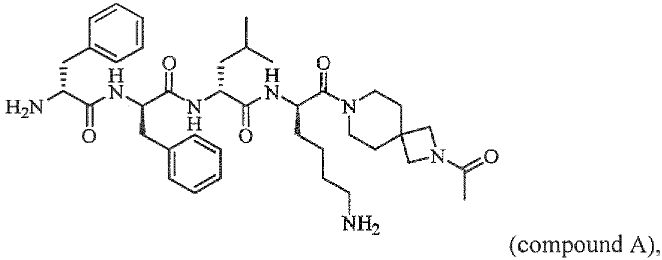 and
b) an absorption enhancer wherein the absorption enhancer is selected from one or more of N-[8-(2-hydroxybenzoyl)amino]octanoic acid or a pharmaceutically acceptable salt thereof, 4-[(4-chloro-2-hydroxybenzoyl)amino]butyric acid or a pharmaceutically acceptable salt thereof, lauroyl-L-carnitine or a hydrochloride thereof, sodium caprylate, sodium caprate, capric acid, caprylocaproyl macrogolglyceride; preferably, the pharmaceutically acceptable salt is selected from sodium salt, potassium salt or calcium salt.

In some embodiments of the present invention, the oral pharmaceutical composition comprises:
a) compound A: 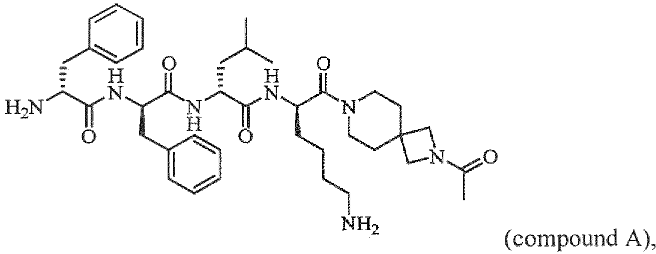 and
b) an absorption enhancer selected from N-[8-(2-hydroxybenzoyl)amino]octanoic acid or a pharmaceutically acceptable salt thereof.

In some embodiments of the present invention, the weight ratio of compound A to N-[8-(2-hydroxybenzoyl)amino]octanoic acid or a pharmaceutically acceptable salt thereof is 1 : 20-1 : 80, preferably 1 : 40-1 : 60, further preferably 1 : 40.

In some embodiments of the present invention, the weight ratio of compound A to N-[8-(2-hydroxybenzoyl)amino]octanoic acid or a pharmaceutically acceptable salt thereof is 1 : 20.

In some embodiments of the present invention, the weight ratio of compound A to N-[8-(2-hydroxybenzoyl)amino]octanoic acid or a pharmaceutically acceptable salt thereof is 1 : 30.

In some embodiments of the present invention, the weight ratio of compound A to N-[8-(2-hydroxybenzoyl)amino]octanoic acid or a pharmaceutically acceptable salt thereof is 1 : 40.

In some embodiments of the present invention, the weight ratio of compound A to N-[8-(2-hydroxybenzoyl)amino]octanoic acid or a pharmaceutically acceptable salt thereof is 1 : 80.

In some embodiments of the present invention, the absorption enhancer is N-[8-(2-hydroxybenzoyl)amino]sodium caprylate.

In some embodiments of the present invention, the absorption enhancer is N-[8-(2-hydroxybenzoyl)amino]potassium caprylate.

In some embodiments of the present invention, the absorption enhancer is N-[8-(2-hydroxybenzoyl)amino]calcium caprylate.

In some embodiments of the present invention, the oral pharmaceutical composition comprises:
a) compound A: 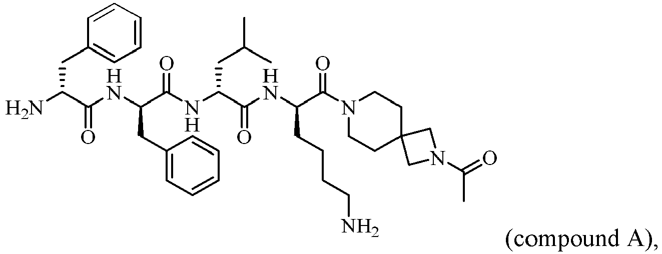 and
b) an absorption enhancer selected from N-[8-(2-hydroxybenzoyl)amino]octanoic acid or a pharmaceutically acceptable salt thereof;
c) one or more non-active ingredients selected from:
   (1) a filler, which is preferably selected from one or more of microcrystalline cellulose, lactose, mannitol, anhydrous calcium hydrogenphosphate, pregelatinized starch, calcium hydrogenphosphate dihydrate;
   (2) a binder, which is preferably selected from one or more of povidone, copovidone, a cellulose derivative, wherein the cellulose derivative is selected from one or more of hydroxypropylcellulose, hypromellose, methyl cellulose;
   (3) a disintegrant, which is preferably selected from one or more of crospovidone, low substituted hydroxypropyl cellulose, croscarmellose sodium, sodium carboxymethyl starch;
   (4) a pH regulator, which is preferably selected from one or more of citric acid, anhydrous citric acid, tartaric acid, fumaric acid, sodium citrate, calcium hydrogenphosphate, calcium carbonate;
   (5) a surfactant, which is preferably selected from one or more of polyethylene glycol, poloxamer, phospholipid, Tween 80, Span 40, propylene glycol monolaurate, sodium dodecyl sulfate;
   (6) a lubricant, which is preferably selected from one or more of magnesium stearate, stearic acid, sodium stearyl fumarate, glyceryl behenate.

In some embodiments of the present invention, the oral pharmaceutical composition comprises:
a) compound A: 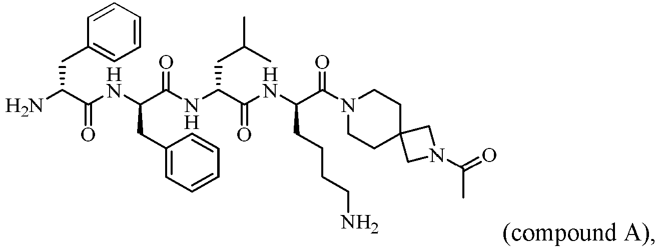 and
b) an absorption enhancer selected from 4-[(4-chloro-2-hydroxybenzoyl)amino]butyric acid or a pharmaceutically acceptable salt thereof.

In some embodiments of the present invention, the weight ratio of compound A to 4-[(4-chloro-2-hydroxy-benzoyl)amino]butyric acid or a pharmaceutically acceptable salt thereof is 1 : 20-1 : 200, preferably 1 : 40-1 : 80, further preferably 1 : 40.

In some embodiments of the present invention, the weight ratio of compound A to 4-[(4-chloro-2-hydroxy-benzoyl)amino]butyric acid or a pharmaceutically acceptable salt thereof is 1 : 20.

In some embodiments of the present invention, the weight ratio of compound A to 4-[(4-chloro-2-hydroxy-benzoyl)amino]butyric acid or a pharmaceutically acceptable salt thereof is 1 : 40.

In some embodiments of the present invention, the weight ratio of compound A to 4-[(4-chloro-2-hydroxy-benzoyl)amino]butyric acid or a pharmaceutically acceptable salt thereof is 1 : 80.

In some embodiments of the present invention, the weight ratio of compound A to 4-[(4-chloro-2-hydroxy-benzoyl)amino]butyric acid or a pharmaceutically acceptable salt thereof is 1 : 100.

In some embodiments of the present invention, the weight ratio of compound A to 4-[(4-chloro-2-hydroxy-benzoyl)amino]butyric acid or a pharmaceutically acceptable salt thereof is 1 : 200.

In some embodiments of the present invention, the weight ratio of compound A to 4-[(4-chloro-2-hydroxy-benzoyl)amino]butyric acid or a pharmaceutically acceptable salt thereof is 3 : 200.

In some embodiments of the present invention, the absorption enhancer is 4-[(4-chloro-2-hydroxy-benzoyl)amino]sodium butyrate.

In some embodiments of the present invention, the absorption enhancer is 4-[(4-chloro-2-hydroxy-benzoyl)amino]potassium butyrate.

In some embodiments of the present invention, the absorption enhancer is 4-[(4-chloro-2-hydroxy-benzoyl)amino]calcium butyrate.

In some embodiments of the present invention, the oral pharmaceutical composition comprises:
a) compound A: 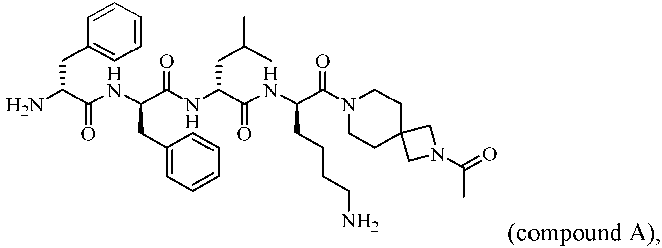 and
b) an absorption enhancer selected from 4-[(4-chloro-2-hydroxybenzoyl)amino]butyric acid or a pharmaceutically acceptable salt thereof;
c) one or more non-active ingredients selected from:
   (1) a filler, which is preferably selected from one or more of microcrystalline cellulose, lactose, mannitol, anhydrous calcium hydrogenphosphate, pregelatinized starch, calcium hydrogenphosphate dihydrate;
   (2) a binder, which is preferably selected from one or more of povidone, copovidone, a cellulose derivative, wherein the cellulose derivative is selected from one or more of hydroxypropylcellulose, hypromellose, methyl cellulose;
   (3) a disintegrant, which is preferably selected from one or more of crospovidone, low substituted hydroxypropyl cellulose, croscarmellose sodium, sodium carboxymethyl starch;
   (4) a pH regulator, which is preferably selected from one or more of citric acid, anhydrous citric acid, tartaric acid, fumaric acid, sodium citrate, calcium hydrogenphosphate, calcium carbonate;
   (5) a surfactant, which is preferably selected from one or more of polyethylene glycol, poloxamer, phospholipid, Tween 80, Span 40, propylene glycol monolaurate, sodium dodecyl sulfate;
   (6) a lubricant, which is preferably selected from one or more of magnesium stearate, stearic acid, sodium stearyl fumarate, glyceryl behenate.

In some embodiments of the present invention, the lubricant is magnesium stearate.

In some embodiments of the present invention, the filler is microcrystalline cellulose and/or anhydrous calcium hydrogenphosphate.

In some embodiments of the present invention, the binder is povidone.

In some embodiments of the present invention, the oral pharmaceutical composition comprises:
a) compound A: 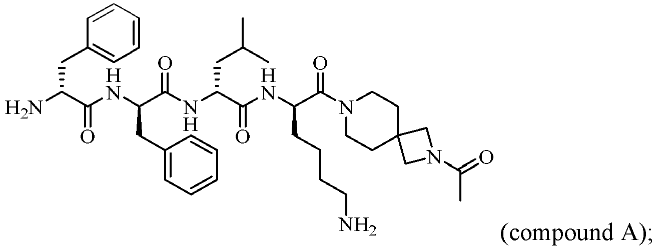
b) 4-[(4-chloro-2-hydroxy-benzoyl)amino]butyric acid or a pharmaceutically acceptable salt thereof;
c) a lubricant.

In some embodiments of the present invention, the weight ratio of compound A : 4-[(4-chloro-2-hydroxy-benzoyl)amino]butyric acid or a pharmaceutically acceptable salt thereof : the lubricant is 1 : (20-200) : (0.2-2).

In some embodiments of the present invention, the weight ratio of compound A : 4-[(4-chloro-2-hydroxy-benzoyl)amino]butyric acid or a pharmaceutically acceptable salt thereof : the lubricant is 1 : 200 : 2.

In some embodiments of the present invention, the oral pharmaceutical composition comprises:
a) compound A: 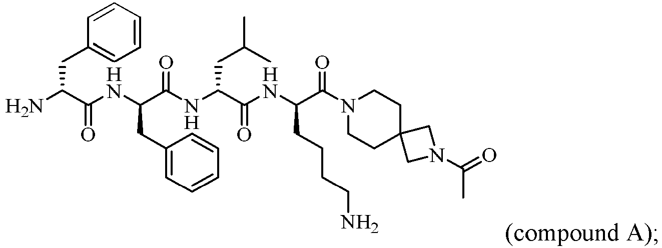
b) 4-[(4-chloro-2-hydroxy-benzoyl)amino]butyric acid or a pharmaceutically acceptable salt thereof;
c) magnesium stearate.

In some embodiments of the present invention, the weight ratio of compound A : 4-[(4-chloro-2-hydroxy-benzoyl)amino]butyric acid or a pharmaceutically acceptable salt thereof : magnesium stearate is 1 : (20-200) : (0.2-2).

In some embodiments of the present invention, the weight ratio of compound A : 4-[(4-chloro-2-hydroxy-benzoyl)amino]butyric acid or a pharmaceutically acceptable salt thereof : magnesium stearate is 1 : 200 : 2.

In some embodiments of the present invention, the oral pharmaceutical composition comprises:
a) compound A: 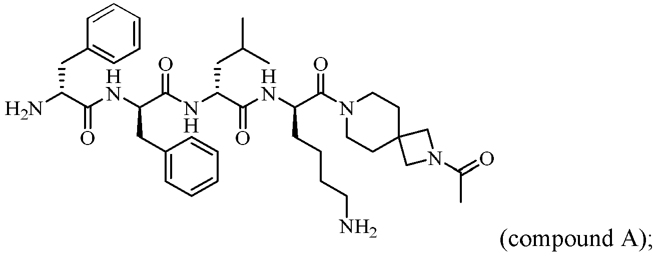
b) 4-[(4-chloro-2-hydroxy-benzoyl)amino]butyric acid or a pharmaceutically acceptable salt thereof;
c) a filler;
d) a binder.

In some embodiments of the present invention, the weight ratio of compound A : 4-[(4-chloro-2-hydroxy-benzoyl)amino]butyric acid or a pharmaceutically acceptable salt thereof : the filler : the binder is 1 : (20-200) : 200 : 5.

In some embodiments of the present invention, the weight ratio of compound A : 4-[(4-chloro-2-hydroxy-benzoyl)amino]butyric acid or a pharmaceutically acceptable salt thereof : the filler : the binder is 1 : 200 : 200 : 5.

In some embodiments of the present invention, the oral pharmaceutical composition comprises:
a) compound A: 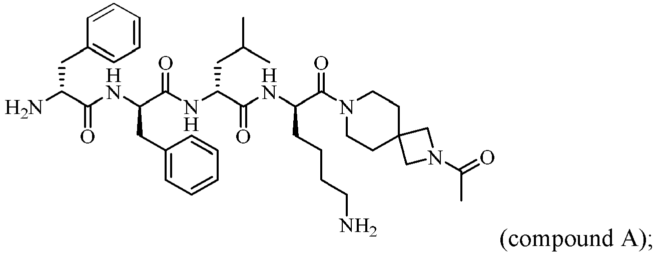
b) 4-[(4-chloro-2-hydroxy-benzoyl)amino]butyric acid or a pharmaceutically acceptable salt thereof;
c) microcrystalline cellulose;
d) povidone.

In some embodiments of the present invention, the weight ratio of compound A : 4-[(4-chloro-2-hydroxy-benzoyl)amino]butyric acid or a pharmaceutically acceptable salt thereof : microcrystalline cellulose : povidone is 1 : (20-200) : 200 : 5.

In some embodiments of the present invention, the weight ratio of compound A : 4-[(4-chloro-2-hydroxy-benzoyl)amino]butyric acid or a pharmaceutically acceptable salt thereof : microcrystalline cellulose : povidone is 1 : 200 : 200 : 5.

In some embodiments of the present invention, the oral pharmaceutical composition comprises:
a) compound A: 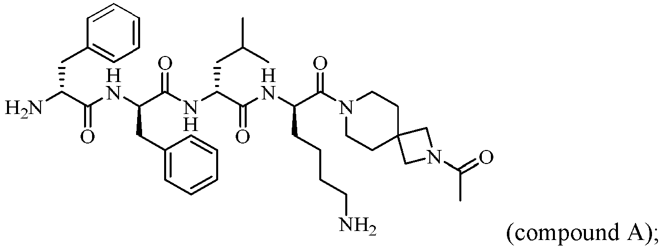
b) 4-[(4-chloro-2-hydroxy-benzoyl)amino]butyric acid or a pharmaceutically acceptable salt thereof;
c) a filler;
d) a binder;
e) a lubricant.

In some embodiments of the present invention, the weight ratio of compound A : 4-[(4-chloro-2-hydroxy-benzoyl)amino]butyric acid or a pharmaceutically acceptable salt thereof : the filler : the binder : the lubricant is 1 : (20-200) : 200 : 5 : (1-5).

In some embodiments of the present invention, the weight ratio of compound A : 4-[(4-chloro-2-hydroxy-benzoyl)amino]butyric acid or a pharmaceutically acceptable salt thereof : the filler : the binder : the lubricant is 1 : 200 : 200 : 5 : (4-5), for example 1 : 200 : 200 : 5 : 4.06.

In some embodiments of the present invention, the weight ratio of compound A : 4-[(4-chloro-2-hydroxy-benzoyl)amino]butyric acid or a pharmaceutically acceptable salt thereof : the filler : the binder : the lubricant is 3 : 200 : 200 : 5 : 1.

In some embodiments of the present invention, the weight ratio of compound A : 4-[(4-chloro-2-hydroxy-benzoyl)amino]butyric acid or a pharmaceutically acceptable salt thereof : the filler : the binder : the lubricant is 3 : 200 : 200 : 5 : 4.

In some embodiments of the present invention, the oral pharmaceutical composition comprises:
a) compound A: 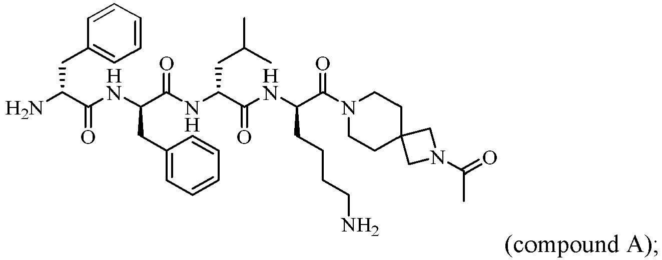
b) 4-[(4-chloro-2-hydroxy-benzoyl)amino]butyric acid or a pharmaceutically acceptable salt thereof;
c) microcrystalline cellulose;
d) povidone;
e) magnesium stearate.

In some embodiments of the present invention, the weight ratio of compound A : 4-[(4-chloro-2-hydroxy-benzoyl)amino]butyric acid or a pharmaceutically acceptable salt thereof : microcrystalline cellulose : povidone : magnesium stearate is (1-3) : (20-200) : 200 : 5 : (1-5).

In some embodiments of the present invention, the weight ratio of compound A : 4-[(4-chloro-2-hydroxy-benzoyl)amino]butyric acid or a pharmaceutically acceptable salt thereof : microcrystalline cellulose : povidone : magnesium stearate is 1 : 200 : 200 : 5 : (4-5), for example 1 : 200 : 200 : 5 : 4.06.

In some embodiments of the present invention, the weight ratio of compound A : 4-[(4-chloro-2-hydroxy-benzoyl)amino]butyric acid or a pharmaceutically acceptable salt thereof : microcrystalline cellulose : povidone : magnesium stearate is 3 : 200 : 200 : 5 : 4.

In some embodiments of the present invention, the oral pharmaceutical composition comprises:
a) compound A: 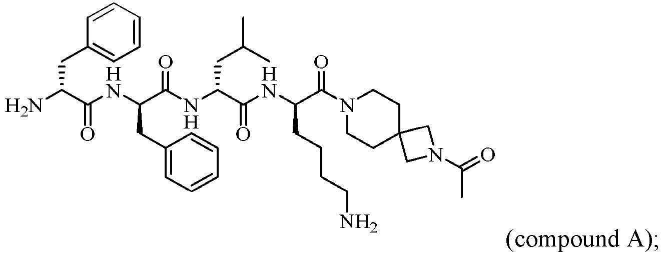
b) 4-[(4-chloro-2-hydroxy-benzoyl)amino]butyric acid or a pharmaceutically acceptable salt thereof;
c) povidone;
d) anhydrous calcium hydrogenphosphate;
e) magnesium stearate.

In some embodiments of the present invention, the weight ratio of compound A : 4-[(4-chloro-2-hydroxy-benzoyl)amino]butyric acid or a pharmaceutically acceptable salt thereof : povidone : anhydrous calcium hydrogenphosphate : magnesium stearate is (1-3) : (20-200) : 5 : 200 : (1-5).

In some embodiments of the present invention, the weight ratio of compound A : 4-[(4-chloro-2-hydroxy-benzoyl)amino]butyric acid or a pharmaceutically acceptable salt thereof : povidone : anhydrous calcium hydrogenphosphate : magnesium stearate is 3 : 200 : 5 : 200 : 4.

In some embodiments of the present invention, the oral pharmaceutical composition comprises:
a) compound A: 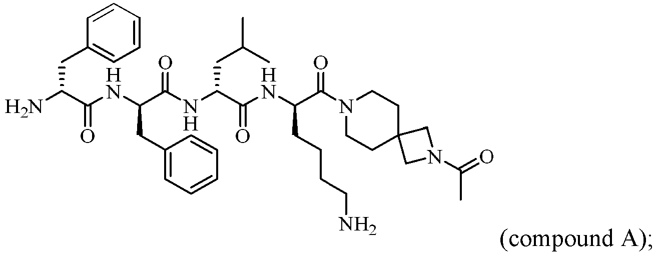
b) 4-[(4-chloro-2-hydroxy-benzoyl)amino]butyric acid or a pharmaceutically acceptable salt thereof;
c) a surfactant;
d) a filler;
e) a lubricant.

In some embodiments of the present invention, the weight ratio of compound A : 4-[(4-chloro-2-hydroxy-benzoyl)amino]butyric acid or a pharmaceutically acceptable salt thereof : the surfactant : the filler : the lubricant is (1-3) : (20-200) : (3-9) : 200 : (1-5), for example 3 : 200 : 6 : 200 : 1.

In some embodiments of the present invention, the oral pharmaceutical composition comprises:
a) compound A: 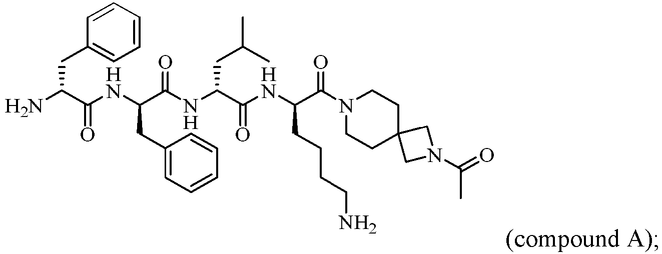
b) 4-[(4-chloro-2-hydroxy-benzoyl)amino]butyric acid or a pharmaceutically acceptable salt thereof;
c) propylene glycol monolaurate and/or polyethylene glycol (for example polyethylene glycol 300);
d) anhydrous calcium hydrogenphosphate;
e) magnesium stearate.

In some embodiments of the present invention, the oral pharmaceutical composition comprises:
a) compound A: 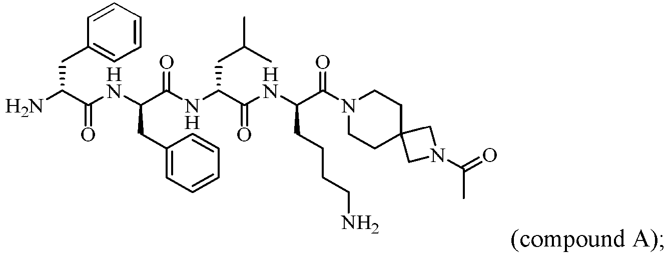
b) 4-[(4-chloro-2-hydroxy-benzoyl)amino]butyric acid or a pharmaceutically acceptable salt thereof;
c) propylene glycol monolaurate;
d) polyethylene glycol 300;
e) anhydrous calcium hydrogenphosphate;
f) magnesium stearate.

In some embodiments of the present invention, the weight ratio of compound A : 4-[(4-chloro-2-hydroxy-benzoyl)amino]butyric acid or a pharmaceutically acceptable salt thereof : propylene glycol monolaurate : polyethylene glycol 300 : anhydrous calcium hydrogenphosphate : magnesium stearate is (1-3) : (20-200) : (3-9) : (3-9) : 200 : (1-5), for example 3 : 200 : 3 : 3 : 200 : 1.

In some embodiments of the present invention, the oral pharmaceutical composition comprises:
a) compound A: 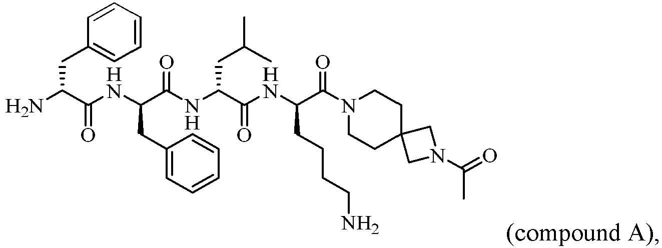 and
b) an absorption enhancer selected from lauroyl-L-carnitine or a hydrochloride thereof.

In some embodiments of the present invention, the weight ratio of compound A to lauroyl-L-carnitine or a hydrochloride thereof is 1 : 10-1 : 150, for example 1 : 10, 1 : 15, 1 : 20, 1 : 30, 1 : 50, 1 : 75, 1 : 100 or 1 : 150.

In some embodiments of the present invention, the oral pharmaceutical composition comprises:
a) compound A: 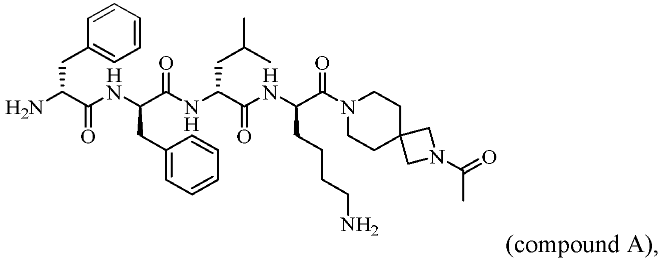 and
b) an absorption enhancer selected from lauroyl-L-carnitine.

In some embodiments of the present invention, the weight ratio of compound A to lauroyl-L-carnitine is 1 : 10-1 : 50, preferably 1 : 10-1 : 20, further preferably 1 : 10.

In some embodiments of the present invention, the weight ratio of compound A to lauroyl-L-carnitine is 1 : 10.

In some embodiments of the present invention, the weight ratio of compound A to lauroyl-L-carnitine is 1 : 20.

In some embodiments of the present invention, the weight ratio of compound A to lauroyl-L-carnitine is 1 : 50.

In some embodiments of the present invention, the oral pharmaceutical composition comprises:
a) compound A: 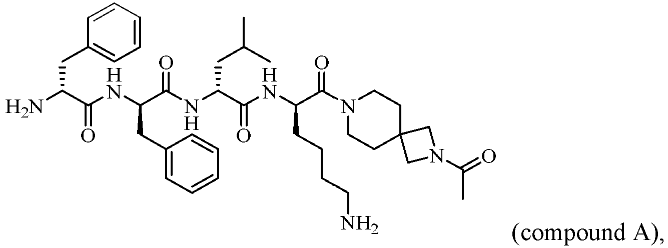 and
b) an absorption enhancer selected from lauroyl-L-carnitine hydrochloride.

In some embodiments of the present invention, the weight ratio of compound A to lauroyl-L-carnitine hydrochloride is 1 : 15-1 : 150, preferably lauroyl-L-carnitine hydrochloride is used in an amount of 75-150 mg, further preferably lauroyl-L-carnitine hydrochloride is used in an amount of 75 mg.

In some embodiments of the present invention, the weight ratio of compound A to lauroyl-L-carnitine hydrochloride is 1 : 15.

In some embodiments of the present invention, the weight ratio of compound A to lauroyl-L-carnitine hydrochloride is 1 : 20.

In some embodiments of the present invention, the weight ratio of compound A to lauroyl-L-carnitine hydrochloride is 1 : 30.

In some embodiments of the present invention, the weight ratio of compound A to lauroyl-L-carnitine hydrochloride is 1 : 75.

In some embodiments of the present invention, the weight ratio of compound A to lauroyl-L-carnitine hydrochloride is 1 : 100.

In some embodiments of the present invention, the weight ratio of compound A to lauroyl-L-carnitine hydrochloride is 1 : 150.

In some embodiments of the present invention, when the absorption enhancer in the oral pharmaceutical composition is selected from lauroyl-L-carnitine or a hydrochloride thereof, the pharmaceutical composition does not comprise enteric coated excipients.

In some embodiments of the present invention, when the absorption enhancer in the oral pharmaceutical composition is selected from lauroyl-L-carnitine or a hydrochloride thereof, the pharmaceutical composition is not in the form of an enteric coated tablet or an enteric coated capsule.

In some embodiments of the present invention, when the absorption enhancer in the oral pharmaceutical composition is selected from lauroyl-L-carnitine or a hydrochloride thereof, the pharmaceutical composition is in the form of a gastric coated capsule, gastric coated tablet or gastric coated granule.

In some embodiments of the present invention, the oral pharmaceutical composition comprises:
a) compound A: 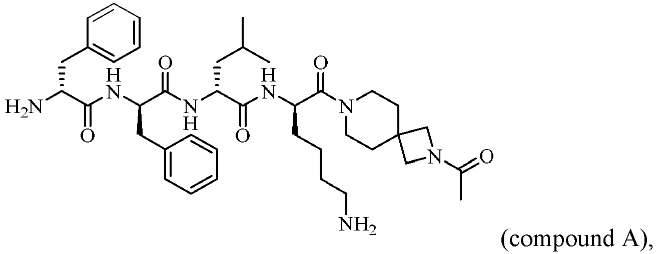 and
b) an absorption enhancer selected from lauroyl-L-carnitine or a hydrochloride thereof;
c) one or more non-active ingredients selected from:
   (1) a filler, which is preferably selected from one or more of microcrystalline cellulose, lactose, mannitol, anhydrous calcium hydrogenphosphate, pregelatinized starch, calcium hydrogenphosphate dihydrate;
   (2) a binder, which is preferably selected from one or more of povidone, copovidone, a cellulose derivative, the cellulose derivative is selected from one or more of hydroxypropylcellulose, hypromellose, methyl cellulose;
   (3) a disintegrant, which is preferably selected from one or more of crospovidone, low substituted hydroxypropyl cellulose, croscarmellose sodium, sodium carboxymethyl starch;
   (4) a pH regulator, which is preferably selected from one or more of citric acid, anhydrous citric acid, tartaric acid, fumaric acid, sodium citrate, calcium hydrogenphosphate, calcium carbonate;
   (5) a surfactant, which is preferably selected from one or more of polyethylene glycol, poloxamer, phospholipid, Tween 80, Span 40, propylene glycol monolaurate, sodium dodecyl sulfate;
   (6) a lubricant, which is preferably selected from one or more of magnesium stearate, stearic acid, sodium stearyl fumarate, glyceryl behenate.

In some embodiments of the present invention, the oral pharmaceutical composition comprises:
a) compound A: 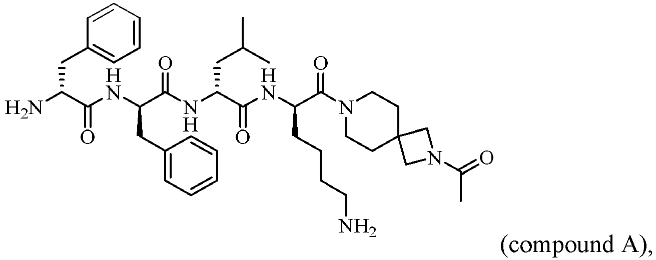 and
b) lauroyl-L-carnitine;
c) a pH regulator.

In some embodiments of the present invention, the weight ratio of compound A : lauroyl-L-carnitine : the pH regulator is 1 : (10-50) : 24.

In some embodiments of the present invention, the oral pharmaceutical composition comprises:
a) compound A: 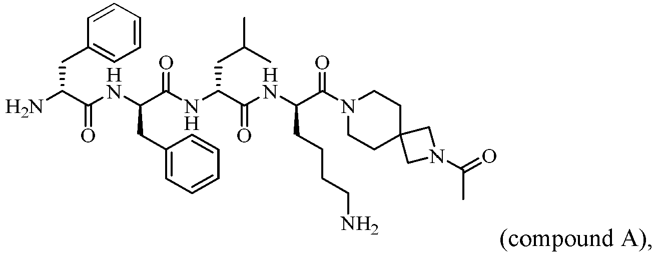 and
b) lauroyl-L-carnitine;
c) citric acid.

In some embodiments of the present invention, the weight ratio of compound A : lauroyl-L-carnitine : citric acid is 1 : (10-50) : 24.

In some embodiments of the present invention, the weight ratio of compound A : lauroyl-L-carnitine : citric acid is 1 : 50 : 24.

In some embodiments of the present invention, the oral pharmaceutical composition comprises:
a) compound A: 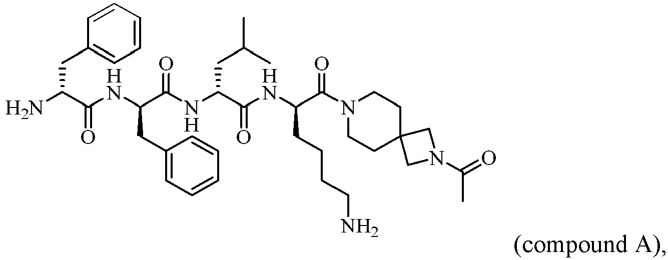 and
b) an absorption enhancer selected from sodium caprate.

In some embodiments of the present invention, the weight ratio of compound A to sodium caprate is 1 : 50-1 : 200, preferably 1 : 100-1 : 200, most preferably 1 : 100.

In some embodiments of the present invention, the weight ratio of compound A to sodium caprate is 1 : 50.

In some embodiments of the present invention, the weight ratio of compound A to sodium caprate is 1 : 100.

In some embodiments of the present invention, the weight ratio of compound A to sodium caprate is 1 : 200.

In some embodiments of the present invention, the oral pharmaceutical composition comprises:
a) compound A: 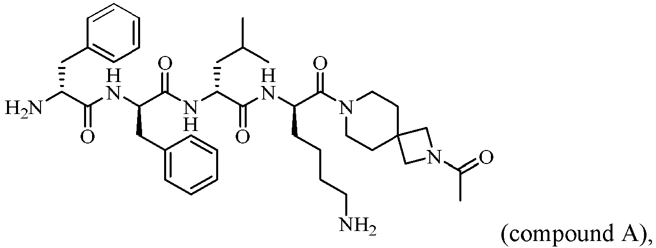 and
b) an absorption enhancer selected from sodium caprate;
c) one or more non-active ingredients selected from:
   (1) a filler, which is preferably selected from one or more of microcrystalline cellulose, lactose, mannitol, anhydrous calcium hydrogenphosphate, pregelatinized starch, calcium hydrogenphosphate dihydrate;
   (2) a binder, which is preferably selected from one or more of povidone, copovidone, a cellulose derivative, wherein the cellulose derivative is selected from one or more of hydroxypropylcellulose, hypromellose, methyl cellulose;
   (3) a disintegrant, which is preferably selected from one or more of crospovidone, low substituted hydroxypropyl cellulose, croscarmellose sodium, sodium carboxymethyl starch;
   (4) a glyceride, which is preferably selected from one or more of monocaprylin, tricaprylin.
   (5) a surfactant, which is preferably selected from one or more of caprylocaproyl macrogolglyceride, polyethylene glycol, poloxamer, phospholipid, Tween 80, Span 40, propylene glycol monolaurate, sodium dodecyl sulfate;
   (6) a lubricant, which is preferably selected from one or more of magnesium stearate, stearic acid, sodium stearyl fumarate, glyceryl behenate.

In some embodiments of the present invention, the binder is povidone.

In some embodiments of the present invention, the oral pharmaceutical composition comprises:
a) compound A: 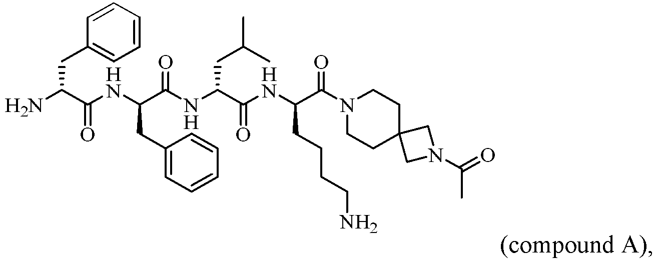 and
b) sodium caprate;
c) povidone.

In some embodiments of the present invention, the weight ratio of compound A : sodium caprate : povidone is 1 : (50-200) : 50, for example 1 : 50 : 50, 1 : 100 : 50 or 1 : 200 : 50.

In some embodiments of the present invention, the oral pharmaceutical composition comprises:
a) compound A: 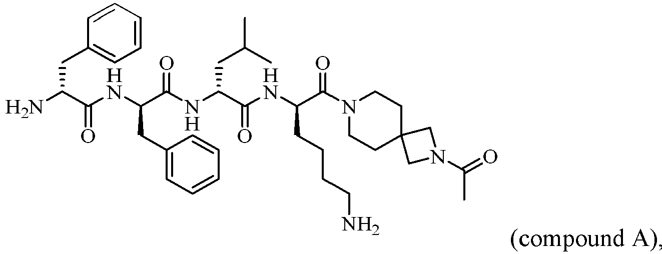 and
b) an absorption enhancer selected from capric acid;
c) one or more non-active ingredients selected from:
   (1) a filler, preferably the filler is selected from one or more of microcrystalline cellulose, lactose, mannitol, anhydrous calcium hydrogenphosphate, pregelatinized starch, calcium hydrogenphosphate dihydrate;
   (2) a binder, which is preferably selected from one or more of povidone, copovidone, a cellulose derivative, wherein the cellulose derivative is selected from one or more of hydroxypropylcellulose, hypromellose, methyl cellulose;
   (3) a disintegrant, which is preferably selected from one or more of crospovidone, low substituted hydroxypropyl cellulose, croscarmellose sodium, sodium carboxymethyl starch;
   (4) a glyceride, which is preferably selected from one or more of monocaprylin, tricaprylin. (5) a surfactant, preferably the surfactant is selected from one or more of caprylocaproyl macrogolglyceride, polyethylene glycol, poloxamer, phospholipid, Tween 80, Span 40, propylene glycol monolaurate, sodium dodecyl sulfate;
   (6) a lubricant, which is preferably selected from one or more of magnesium stearate, stearic acid, sodium stearyl fumarate, glyceryl behenate.

In some embodiments of the present invention, the oral pharmaceutical composition comprises:
a) compound A: 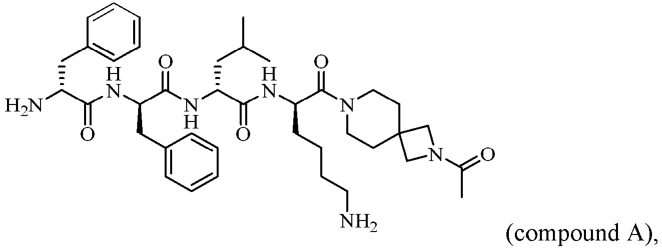 and
b) an absorption enhancer selected from caprylocaproyl macrogolglyceride.

In some embodiments of the present invention, the weight ratio of compound A to caprylocaproyl macrogolglyceride is 1 : 600-1 : 3600, preferably 1 : 2400-1 : 3600, most preferably 1 : 2400.

In some embodiments of the present invention, the weight ratio of compound A to caprylocaproyl macrogolglyceride is 1 : 600.

In some embodiments of the present invention, the weight ratio of compound A to caprylocaproyl macrogolglyceride is 1 : 1200.

In some embodiments of the present invention, the weight ratio of compound A to caprylocaproyl macrogolglyceride is 1 : 1800.

In some embodiments of the present invention, the weight ratio of compound A to caprylocaproyl macrogolglyceride is 1 : 2400.

In some embodiments of the present invention, the weight ratio of compound A to caprylocaproyl macrogolglyceride is 1 : 3600.

In some embodiments of the present invention, the oral pharmaceutical composition comprises:
a) compound A: 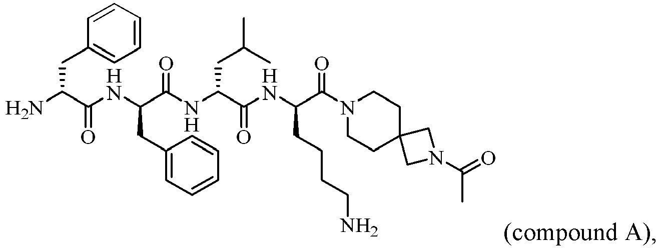 and
b) an absorption enhancer selected from caprylocaproyl macrogolglyceride;
c) one or more non-active ingredients selected from:
   (1) a hydrophobic medium, which is preferably selected from one or more of coconut oil, castor oil, olive oil;
   (2) a surfactant, which is preferably selected from one or more of polyethylene glycol, poloxamer, phospholipid, Tween 80, Span 40, propylene glycol monolaurate, sodium dodecyl sulfate;
   (3) a glyceride, which is preferably selected from one or more of monocaprylin, tricaprylin.

In some embodiments of the present invention, the oral pharmaceutical composition comprises:
a) compound A: 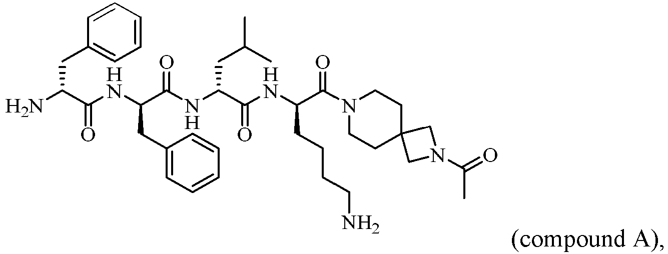 and
b) an absorption enhancer selected from caprylocaproyl macrogolglyceride;
c) one or more non-active ingredients selected from:
   (1) a hydrophobic medium, which is preferably selected from one or more of coconut oil, castor oil, olive oil;
   (2) a surfactant, which is preferably selected from one or more of polyethylene glycol, poloxamer, phospholipid, Tween 80, Span 40, propylene glycol monolaurate, sodium dodecyl sulfate;
   (3) a glyceride other than caprylocaproyl macrogolglyceride, preferably the glyceride is selected from one or more of monocaprylin, tricaprylin.

In some embodiments of the present invention, the oral pharmaceutical composition comprises:
a) compound A: 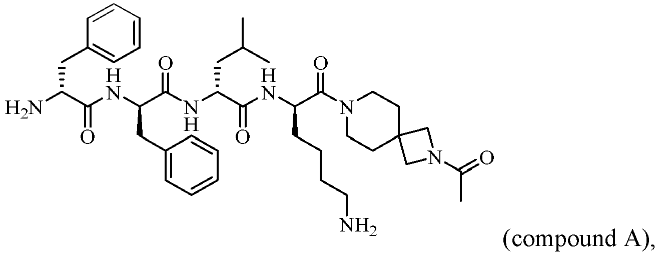 and
b) caprylocaproyl macrogolglyceride;
c) capric acid.

In some embodiments of the present invention, the weight ratio of compound A : caprylocaproyl macrogolglyceride : capric acid is 1 : (3000-6000) : (50-200), for example 1 : 3000 : (50-100) or 1 : (4000-6000) : 200, for example 1 : 3000 : 50, 1 : 3000 : 75, 1 : 3000 : 100, 1 : 4000 : 200 or 1 : 6000 : 200.

In some embodiments of the present invention, the oral pharmaceutical composition is in the form of a solution, a suspension, a granule, a powder, a capsule, a tablet or other oral dosage forms.

In some embodiments of the present invention, the oral pharmaceutical composition is in the form of a gastric coated capsule, a gastric coated tablet or a gastric coated granule.

In some embodiments of the present invention, compound (A) is present in the oral pharmaceutical composition at a therapeutically effective amount or unit dose. In some embodiments of the present invention, compound (A) is present in the oral pharmaceutical composition at a does of less than 1 mg, 1-3 mg, 3-5 mg, 5-10 mg, 10-25 mg, 25-32 mg, 32-100 mg, or more than 100 mg.

In some embodiments of the present invention, the above-mentioned oral pharmaceutical composition is prepared as follows: compound A, the absorption enhancer and other non-active ingredients are mixed directly, and then filled into capsules or compressed into tablets.

In some embodiments of the present invention, the above-mentioned oral pharmaceutical composition is prepared as follows: compound A, the absorption enhancer and other non-active ingredients are wet granulated and then filled into capsules or compressed into tablets.

In some embodiments of the present invention, the above-mentioned oral pharmaceutical composition is prepared as follows: compound A, the absorption enhancer and the hydrophilic non-active ingredients are wet granulated, then dispersed in a hydrophobic medium, and filled into capsules.

In the present invention, the oral pharmaceutical composition can also be prepared as a solution, a suspension, a granule, a powder or other oral dosage forms using a conventional preparation process.

In the present invention, the capsule may be a hard capsule or a soft capsule.

The present invention also provides the use of the oral pharmaceutical composition in preparing a drug for treating diseases or conditions related to the κ-opioid receptor.

The present invention also provides a method for treating diseases or conditions related to the x-opioid receptor in a subject, comprising administering the oral pharmaceutical composition of any one of the foregoing embodiments to the subject.

In some embodiments of the present invention, the diseases or conditions related to the κ-opioid receptor are selected from pain, inflammation, itching, edema, hyponatremia, hypokalemia, intestinal obstruction, cough and glaucoma.

In some embodiments of the present invention, the pain is selected from neuropathic pain, somatic pain, visceral pain and skin pain.

In some embodiments of the present invention, the diseases or conditions are selected from arthritis pain, kidney stone pain, hysterotrismus, dysmenorrhea, endometriosis, dyspepsia, pain after surgery, pain after medical treatment, ocular pain, otitis pain, breakthrough cancer pain, and pain associated with GI disorders (gastrointestinal disorders).

In one embodiment of the present invention, the oral pharmaceutical composition can be used for treating acute and chronic pain and itching.

In certain embodiments, the oral pharmaceutical composition is administered to the subject 1, 2, or 3 times daily.

In certain embodiments, the subject is a mammal, such as a bovine, an equine, a porcine, a canine, a feline, a rodent, a primate animal, wherein, particularly preferred subjects are humans.

### Detailed Description of Embodiments

Unless stated to the contrary, the terms used in the description and claims have the following meanings.
(1) SNAC: sodium N-[8-(2-hydroxybenzoyl)amino]octanoate.
(2) 4-CNAB: sodium 4-[(4-chloro-2-hydroxy-benzoyl)amino]butyrate.

The implementation process and beneficial effects of the present invention are described in detail below through specific examples, which are intended to help readers better understand the essence and characteristics of the present invention, and are not intended to limit the scope of implementation of the present invention.

The raw materials/reagents used in the examples are commercially available or self-prepared, and the formulations in the examples are all at single preparation dosage.

Unless otherwise specified, "0# capsule" in the following examples refers to the shell that can be used to prepare gastric coated hard capsules with the specification of 0#.

Compound A was prepared with reference to the method disclosed in WO 2019/015644.

### Example 1

3 mg of compound A was dissolved in physiological saline to prepare 0.06 mg/ml physiological saline solution.

### Example 2

5 mg of compound A and 150 mg of SNAC were mixed uniformly and then filled into 0# capsules.

### Example 3

5 mg of compound A and 100 mg of SNAC were mixed uniformly and then filled into 0# capsules.

### Example 4

5 mg of compound A and 200 mg of SNAC were mixed uniformly and then filled into 0# capsules.

### Example 5

5 mg of compound A and 400 mg of SNAC were mixed uniformly and then filled into 0# capsules.

### Example 6

5 mg of compound A, 50 mg of lauroyl-L-carnitine and 120 mg of citric acid were mixed uniformly and then filled into 0# capsules.

### Example 7

5 mg of compound A and 100 mg of 4-CNAB were mixed uniformly and then filled into 0# capsules.

### Example 8

5 mg of compound A and 200 mg of 4-CNAB were mixed uniformly and then filled into 0# capsules.

### Example 9

5 mg of compound A and 400 mg of 4-CNAB were mixed uniformly and then filled into 0# capsules.

### Example 10

1 mg of compound A and 200 mg of 4-CNAB were mixed uniformly and then filled into 0# capsules.

### Example 11

1 mg of compound A, 200 mg of 4-CNAB and 2 mg of magnesium stearate were mixed uniformly and then compressed into tablets.

### Example 12

1 mg of compound A, 200 mg of 4-CNAB, 200 mg of microcrystalline cellulose, and 5 mg of povidone were mixed uniformly and then filled into 0# capsules.

### Example 13

1 mg of compound A, 200 mg of 4-CNAB, 200 mg of microcrystalline cellulose, 5 mg of povidone, and 4.06 mg of magnesium stearate were mixed uniformly and then compressed into tablets.

### Example 14

1 mg of compound A, 200 mg of 4-CNAB, 200 mg of microcrystalline cellulose, and 5 mg of povidone were weighed; compound A and 4-CNAB were dissolved in water, subjected to rotary evaporation, dried, ground and pulverized; and same were mixed with the other non-active ingredients uniformly and then filled into 0# capsules.

### Example 15

1 mg of compound A, 200 mg of 4-CNAB, 200 mg of microcrystalline cellulose, 5 mg of povidone, and 4.06 mg of magnesium stearate were weighed; compound A and 4-CNAB were dissolved in water, subjected to rotary evaporation, dried, ground and pulverized; and same were mixed with the other non-active ingredients and then compressed into tablets.

### Example 16

2 mg of compound A and 200 mg of 4-CNAB were mixed uniformly and then filled into 0# capsules.

### Example 17

3 mg of compound A and 200 mg of 4-CNAB were mixed uniformly and then filled into 0# capsules.

### Example 18

10 mg of compound A and 200 mg of 4-CNAB were mixed uniformly and then filled into 0# capsules.

### Example 19

3 mg of compound A, 200 mg of 4-CNAB, 5 mg of povidone, 200 mg of microcrystalline cellulose, and 4 mg of magnesium stearate were mixed uniformly and then filled into 0# capsules.

### Example 20

3 mg of compound A, 200 mg of 4-CNAB, 5 mg of povidone, 200 mg of anhydrous calcium hydrogenphosphate, and 4 mg of magnesium stearate were mixed uniformly and then filled into 0# capsules.

### Example 21

① 3 mg of compound A and 200 mg of 4-CNAB were mixed uniformly.
② 3 mg of propylene glycol monolaurate and 3 mg of polyethylene glycol 300 were absorbed by and dispersed with 200 mg of anhydrous calcium hydrogenphosphate.
③ The samples from the above two steps were mixed uniformly.
④ 1 mg of magnesium stearate was added, and the mixture was mixed uniformly and then filled into 0# capsules.

### Example 22

100 mg of compound A, 5 g of povidone K12 and 5 g of sodium caprate were completely dissolved in 100 g of purified water, then freeze-dried and passed through a 120-mesh sieve and then filled into 0# capsules.

### Example 23

100 mg of compound A, 5 g of povidone K12 and 10 g of sodium caprate were completely dissolved in 100 g of purified water, then freeze-dried and passed through a 120-mesh sieve and then filled into 0# capsules.

### Example 24

25 mg of compound A, 1.25 g of povidone K12 and 5 g of sodium caprate were completely dissolved in 100 g of purified water, then freeze-dried and passed through a 120-mesh sieve and then filled into 00# capsules.

### Example 25

1 mg of compound A and 0.6 g of caprylocaproyl macrogolglyceride were completely dissolved in purified water to obtain a 10 ml solution.

### Example 26

1 mg of compound A and 1.2 g of caprylocaproyl macrogolglyceride were completely dissolved in purified water to obtain a 10 ml solution.

### Example 27

1 mg of compound A and 1.8 g of caprylocaproyl macrogolglyceride were completely dissolved in purified water to obtain a 10 ml solution.

### Example 28

1 mg of compound A and 2.4 g of caprylocaproyl macrogolglyceride were completely dissolved in purified water to obtain a 10 ml solution.

### Example 29

1 mg of compound A and 3.6 g of caprylocaproyl macrogolglyceride were completely dissolved in purified water to obtain a 10 ml solution.

### Example 30

1 mg of compound A and 0.6 g of caprylocaproyl macrogolglyceride were mixed and suspended uniformly by ultrasonic dispersion, and then filled into 0# capsules.

### Example 31

1 mg of compound A and 1.8 g of caprylocaproyl macrogolglyceride were mixed and suspended uniformly by ultrasonic dispersion, and then filled into 0# capsules.

### Example 32

5 mg of compound A and 3.0 g of caprylocaproyl macrogolglyceride were mixed and suspended uniformly by ultrasonic dispersion, and then filled into 0# capsules.

### Example 33

1 mg of compound A, 3 g of caprylocaproyl macrogolglyceride and 50 mg of sodium caprate were completely dissolved in purified water to obtain a 10 ml solution.

### Example 34

1 mg of compound A, 3 g of caprylocaproyl macrogolglyceride and 75 mg of sodium caprate were completely dissolved in purified water to obtain a 10 ml solution.

### Example 35

1 mg of compound A, 3 g of caprylocaproyl macrogolglyceride and 100 mg of sodium caprate were completely dissolved in purified water to obtain a 10 ml solution.

### Example 36

1 mg of compound A, 4 g of caprylocaproyl macrogolglyceride and 200 mg of mgcapric acid were completely dissolved in purified water to obtain a 10 ml solution.

### Example 37

1 mg of compound A, 6 g of caprylocaproyl macrogolglyceride and 200 mg of mgcapric acid were completely dissolved in purified water to obtain a 10 ml solution.

### Example 38

5 mg of compound A and 50 mg of lauroyl-L-carnitine were filled into 0# capsules.

### Example 39

5 mg of compound A and 100 mg of lauroyl-L-carnitine were filled into 0# capsules.

### Example 40

5 mg of compound A and 250 mg of lauroyl-L-carnitine were filled into 0# capsules.

### Example 41

5 mg of compound A and 100 mg of lauroyl-L-carnitine were filled into 0# enteric coated capsules.

### Example 42

5 mg of compound A and 75 mg of lauroyl-L-carnitine hydrochloride were filled into 0# capsules.

### Example 43

5 mg of compound A and 100 mg of lauroyl-L-carnitine hydrochloride were filled into 0# capsules.

### Example 44

5 mg of compound A and 150 mg of lauroyl-L-carnitine hydrochloride were filled into 0# capsules.

### Example 45

1 mg of compound A and 75 mg of lauroyl-L-carnitine hydrochloride were filled into 0# capsules.

### Example 46

1 mg of compound A and 100 mg of lauroyl-L-carnitine hydrochloride were filled into 0# capsules.

### Example 47

1 mg of compound A and 150 mg of lauroyl-L-carnitine hydrochloride were filled into 0# capsules.

### Example 48

1 mg of compound A and 200 mg of sodium caproate were filled into 0# capsules.

### Example 49

1 mg of compound A and 100 mg of sodium caprylate were filled into 0# capsules.

### Example 50

1 mg of compound A and 200 mg of sodium laurate were filled into 0# capsules.

### Example 51

1 mg of compound A and 200 mg of sodium myristate were filled into 0# capsules.

### Example 52

1 mg of compound A and 200 mg of sodium palmitate were filled into 0# capsules.

### Test example 1

The physiological saline solution of compound A obtained in Example 1 was orally administered at a single dose to male Beagle dogs (3 for each preparation), venous blood was taken at 0, 0.25, 0.5, 1, 2, 4, 6, 8, 10, 12 and 24 h, and the absolute bioavailability (F%) (relative to intravenous dose of compound A) was calculated from the area under the curve obtained from the points where the plasma concentration of compound A was taken as a function of time.

After a single oral administration of the capsules obtained in Example 2 to male Beagle dogs (3 for each preparation) with 5 tablets of the samples for each dog, venous blood was taken at 0, 0.083, 0.25, 0.5, 1, 2, 4, 6, 8 and 24 h, and the absolute bioavailability (F%) (relative to intravenous dose of compound A) was calculated from the area under the curve obtained from the points where the plasma concentration of compound A was taken as a function of time.

**Table 1 Effect of SNAC on absorption of compound A**

| Formula | Cₘₐₓ (ng/ml) | F% |
|---|---|---|
| Example 1 | 2.60 ± 0.49 | 1.81 ± 0.01 |
| Example 2 | 125 ± 47 | 3.46 ± 1.62 |

### Results and discussion:

By means of adding 150 mg of SNAC to the formulations, the bioavailability is significantly improved, and the absolute bioavailability is up to 3.46%.

### Test example 2

After a single oral administration of the capsules obtained in Examples 3-5 to male Beagle dogs (3 for each preparation) with 1 tablet of the samples for each dog, venous blood was taken at 0, 0.083, 0.25, 0.5, 0.75, 1, 2, 4, 8, 10, 12 and 24 h, and the absolute bioavailability (F%) (relative to intravenous dose of compound A) was calculated from the area under the curve obtained from the points where the plasma concentration of compound A was taken as a function of time.

**Table 2 Effect of dosage of SNAC on absorption of compound A**

| Formula | Cₘₐₓ (ng/ml) | F% |
|---|---|---|
| Example 1 | 2.60 ± 0.49 | 1.81 ± 0.01 |
| Example 3 | 6.88 ± 5.8 | 2.18 ± 1.3 |
| Example 4 | 109 ± 56 | 9.82 ± 3.8 |
| Example 5 | 41.2 ± 22 | 5.60 ± 3.3 |

### Results and discussion:

In the case of the weight ratio of compound A to SNAC being in the range of 1 : 20-1 : 80, the bioavailability of compound A can be significantly improved, and the absolute bioavailability is better when the weight ratio thereof is 1 : 40.

### Test example 3

After a single oral administration of the capsules obtained in Example 6 to male Beagle dogs (3 for each preparation) with 5 tablets of the samples for each dog, venous blood was taken at 0, 0.25, 0.5, 1, 2, 4, 6, 8, 12, 24, 28, 48 and 72 h, and the absolute bioavailability (F%) (relative to intravenous dose of compound A) was calculated from the area under the curve obtained from the points where the plasma concentration of compound A was taken as a function of time.

**Table 3 Effect of formula on absorption of compound A**

| Formula | Cₘₐₓ (ng/ml) | F% |
|---|---|---|
| Example 1 | 2.60 ± 0.49 | 1.81 ± 0.01 |
| Example 6 | 451 ± 384 | 6.62 ± 5.3 |

### Results and discussion:

Adding 50 mg of lauroyl-L-carnitine and 120 mg of citric acid to the formulations provides a good absorption promoting effect, and the absolute bioavailability of compound A is up to 6.62%.

### Test example 4

After a single oral administration of the capsules obtained in Examples 7-9 to male Beagle dogs (3 for each preparation) with 1 tablet of the samples for each dog, venous blood was taken at 0, 0.083, 0.25, 0.5, 0.75, 1, 2, 4, 8, 10, 12 and 24 h, and the absolute bioavailability (F%) (relative to intravenous dose of compound A) was calculated from the area under the curve obtained from the points where the plasma concentration of compound A was taken as a function of time.

**Table 4 Effect of dosage of 4-CNAB on absorption of compound A**

| Formula | Cₘₐₓ (ng/ml) | F% |
|---|---|---|
| Example 1 | 2.60 ± 0.49 | 1.81 ± 0.01 |
| Example 7 | 61.7 ± 48 | 4.83 ± 2.7 |
| Example 8 | 166 ± 54 | 10.5 ± 1.6 |
| Example 9 | 80.7 ± 70 | 6.63 ± 4.5 |

### Results and discussion:

In the case of the weight ratio of compound A to 4-CNAB being in the range of 1 : 20-1 : 80, the bioavailability of compound A can be significantly improved, and the absolute bioavailability is better when the weight ratio thereof is 1 : 40.

### Test example 5

After a single oral administration of the preparation samples prepared in Examples 10-15 by different processes to male Beagle dogs (3 for each preparation) with 1 tablet of the samples for each dog, venous blood was taken at 0, 0.083, 0.25, 0.5, 1, 2, 4, 6, 8, 12, 24, 28 and 48 h, and the absolute bioavailability (F%) (relative to intravenous dose of compound A) was calculated from the area under the curve obtained from the points where the plasma concentration of compound A was taken as a function of time.

**Table 5 Effect of different preparation processes on absorption of compound A**

| Formula | Preparation method | Cₘₐₓ (ng/ml) | F% |
|---|---|---|---|
| Example 1 | Dissolving in physiological saline | 2.60 ± 0.49 | 1.81 ± 0.01 |
| Example 10 | Mixing uniformly and then filling into capsules | 15.8 ± 11 | 6.14 ± 4.1 |
| Example 11 | Mixing uniformly and then compressing into tablets | 14.5 ± 15 | 5.57 ± 5.5 |
| Example 12 | Mixing uniformly and then filling into capsules | 24.1 ± 19 | 8.57 ± 7.1 |
| Example 13 | Mixing uniformly and then compressing into tablets | 22.0 ± 7.4 | 7.91 ± 2.9 |
| Example 14 | Compound A and 4-CNAB were dissolved in water, subjected to rotary evaporation, dried, ground and pulverized, and same were mixed with other excipients and then filled into capsules | 18.4 ± 3.2 | 5.70 ± 0.46 |
| Example 15 | Compound A and 4-CNAB were dissolved in water, subjected to rotary evaporation, dried, ground and pulverized, and same were mixed with other excipients and then compressed into tablets | 14.2 ± 3.6 | 6.28 ± 1.9 |

### Results and discussion:

By means of adding 4-CNAB to the formulations, the absolute bioavailability of samples at different dosage forms and prepared by different preparation processes is increased significantly.

### Test example 6

After a single oral administration of the preparation samples obtained in Examples 22-24 to male Beagle dogs (3 for each preparation) with 1 tablet of the samples for each dog, venous blood was taken at 0, 0.25, 0.5, 1, 2, 4, 6, 8, 10, 12 and 24 h, and the absolute bioavailability (F%) (relative to intravenous dose of compound A) was calculated from the area under the curve obtained from the points where the plasma concentration of compound A was taken as a function of time.

**Table 6 Effect of dosage of sodium caprate on absorption of compound A**

| Formula | Cₘₐₓ (ng/ml) | F% |
|---|---|---|
| Example 1 | 2.60 ± 0.49 | 1.81 ± 0.01 |
| Example 22 | 1.53 ± 0.19 | 1.36 ± 0.15 |
| Example 23 | 9.10 ± 3.7 | 5.59 ± 2.0 |
| Example 24 | 15.9 ± 14 | 5.73 ± 3.3 |

### Results and discussion:

In the formulations, the bioavailability of compound A is improved by sodium caprate in a dose-response manner. When the weight ratio of compound A to sodium caprate is 1 : 50, the bioavailability does not change significantly, while the weight ratio of compound A to sodium caprate is 1 : 100 and 1 : 200, the bioavailability is increased significantly.

### Test example 7

After a single oral administration of the preparation samples obtained in Examples 25-29 to male Beagle dogs (3 for each preparation) with 10 ml of the samples for each dog, venous blood was taken at 0, 0.25, 0.5, 1, 2, 4, 6, 8, 10, 12 and 24 h, and the absolute bioavailability (F%) (relative to intravenous dose of compound A) was calculated from the area under the curve obtained from the points where the plasma concentration of compound A was taken as a function of time.

**Table 7 Effect of dosage of caprylocaproyl macrogolglyceride on absorption of compound A**

| Formula | Cₘₐₓ (ng/ml) | F% |
|---|---|---|
| Example 1 | 2.60 ± 0.49 | 1.81 ± 0.01 |
| Example 25 | 20.0 ± 3.4 | 6.97 ± 2.7 |
| Example 26 | 16.3 ± 4.0 | 6.53 ± 1.4 |
| Example 27 | 17.4 ± 7.5 | 6.75 ± 2.6 |
| Example 28 | 20.6 ± 4.9 | 9.38 ± 1.6 |
| Example 29 | 21.9 ± 18 | 7.73 ± 4.6 |

### Results and discussion:

In the formulations, the bioavailability is significantly improved when the weight ratio of compound A to caprylocaproyl macrogolglyceride is 1 : 600-1 : 3600, and the absolute bioavailability is better when the weight ratio is 1 : 2400.

### Test example 8

After a single oral administration of the preparation samples obtained in Examples 25, 27 and 30-32 to male Beagle dogs (3 for each preparation), venous blood was taken at 0, 0.25, 0.5, 1, 2, 4, 6, 8, 10, 12 and 24 h, and the absolute bioavailability (F%) (relative to intravenous dose of compound A) was calculated from the area under the curve obtained from the points where the plasma concentration of compound A was taken as a function of time.

**Table 8 Effect of dosage forms and specification on absorption of compound A**

| Formula | Cₘₐₓ (ng/ml) | F% |
|---|---|---|
| Example 1 | 2.60 ± 0.49 | 1.81 ± 0.01 |
| Example 25 | 20.0 ± 3.4 | 6.97 ± 2.7 |
| Example 30 | 28.0 ± 11 | 8.63 ± 2.5 |
| Example 27 | 17.4 ± 7.5 | 6.75 ± 2.6 |
| Example 31 | 17.8 ± 4.7 | 6.47 ± 1.8 |
| Example 32 | 65.1 ± 40 | 6.97 ± 4.7 |

### Results and discussion:

Both solutions and capsules obtained by adding caprylocaproyl macrogolglyceride to the formulations can significantly improve the bioavailability of compound A, and the improvement levels of the bioavailability of compound A are similar at the specification of 1 mg and 5 mg.

### Test example 9

After a single oral administration of the preparation samples obtained in Examples 33-35 to male Beagle dogs (3 for each preparation) with 10 ml of the samples for each dog, venous blood was taken at 0, 0.25, 0.5, 1, 2, 4, 6, 8, 10, 12 and 24 h, and the absolute bioavailability (F%) (relative to intravenous dose of compound A) was calculated from the area under the curve obtained from the points where the plasma concentration of compound A was taken as a function of time.

**Table 9 Effect of dosage of caprylocaproyl macrogolglyceride and sodium caprate on absorption of compound A**

| Formula | Cₘₐₓ (ng/ml) | F% |
|---|---|---|
| Example 1 | 2.60 ± 0.49 | 1.81 ± 0.01 |
| Example 33 | 17.9 ± 12 | 7.52 ± 5.1 |
| Example 34 | 16.1 ± 7.3 | 6.95 ± 2.6 |
| Example 35 | 23.2 ± 4.5 | 12.2 ± 2.9 |

### Results and discussion:

In the formulations, the bioavailability of compound A is significantly improved when the weight ratio of compound A : caprylocaproyl macrogolglyceride : sodium caprate is 1 : 3000 : 50-100, and the bioavailability is better when the weight ratio is 1 : 3000 : 100.

### Test example 10

After a single oral administration of the preparation samples obtained in Examples 36-37 to male Beagle dogs (3 for each preparation) with 10 ml of the samples for each dog, venous blood was taken at 0, 0.25, 0.5, 1, 2, 4, 6, 8, 10, 12 and 24 h, and the absolute bioavailability (F%) (relative to intravenous dose of compound A) was calculated from the area under the curve obtained from the points where the plasma concentration of compound A was taken as a function of time.

**Table 10 Effect of dosage of caprylocaproyl macrogolglyceride and capric acid on absorption of compound A**

| Formula | Cₘₐₓ (ng/ml) | F% |
|---|---|---|
| Example 1 | 2.60 ± 0.49 | 1.81 ± 0.01 |
| Example 36 | 15.0 ± 5.8 | 6.02 ± 1.7 |
| Example 37 | 15.1 ± 1.7 | 7.86 ± 1.6 |

In the formulations, the bioavailability of compound A is significantly improved when the weight ratio of compound A : caprylocaproyl macrogolglyceride : capric acid is 1 : (4000-6000) : 200, and the bioavailability is better when the weight ratio is 1 : 6000 : 200.

### Test example 11

After a single oral administration of the preparation samples obtained in Examples 43-45 to male Beagle dogs (3 for each preparation) with 1 tablet of the samples for each dog, venous blood was taken at 0, 0.25, 0.5, 1, 2, 4, 6, 8, 10, 12 and 24 h, and the absolute bioavailability (F%) (relative to intravenous dose of compound A) was calculated from the area under the curve obtained from the points where the plasma concentration of compound A was taken as a function of time.

**Table 11 Effect of dosage of lauroyl-L-carnitine on absorption of compound A**

| Formula | Cₘₐₓ (ng/ml) | F% |
|---|---|---|
| Example 1 | 2.60 ± 0.49 | 1.81 ± 0.01 |
| Example 38 | 56.6 ± 82 | 5.33 ± 5.9 |
| Example 39 | 232 ± 124 | 16.7 ± 5.2 |
| Example 40 | 105 ± 103 | 8.92 ± 8.0 |
| Example 41 | 62.8 ± 13 | 4.05 ± 0.48 |

### Results and discussion:

In the formulations, the bioavailability of compound A is significantly improved when the weight ratio of compound A to lauroyl-L-carnitine is 1 : 10-1 : 50, and the bioavailability is better when the weight ratio is 1 : 20.

When the weight ratio of compound A to lauroyl-L-carnitine is 1 : 20, the bioavailability of the gastric coated capsule preparation is 4 times that of the enteric coated capsule preparation.

### Test example 12

After a single oral administration of the preparation samples obtained in Examples 42-47 to male Beagle dogs (3 for each preparation) 1 tablet of the samples for each dog, venous blood was taken at 0, 0.25, 0.5, 1, 2, 4, 6, 8, 10, 12 and 24 h, and the absolute bioavailability (F%) (relative to intravenous dose of compound A) was calculated from the area under the curve obtained from the points where the plasma concentration of compound A was taken as a function of time.

**Table 12 Effect of dosage of lauroyl-L-carnitine hydrochloride on absorption of compound A**

| Formula | Cₘₐₓ (ng/ml) | F% |
|---|---|---|
| Example 1 | 2.60 ± 0.49 | 1.81 ± 0.01 |
| Example 42 | 117 ± 49 | 11.8 ± 6.1 |
| Example 43 | 90.1 ± 107 | 8.32 ± 9.3 |
| Example 44 | 125 ± 33 | 8.30 ± 1.3 |
| Example 45 | 25.4 ± 25 | 12.4 ± 8.9 |
| Example 46 | 16.5 ± 8.3 | 9.17 ± 5.3 |
| Example 47 | 24.1 ± 20 | 6.04 ± 4.1 |

### Results and discussion:

The bioavailability of compound A is significantly improved when the weight ratio of compound A to lauroyl-L-carnitine hydrochloride is 1 : 15-1 : 150, and the bioavailability is better when the weight ratio is 1 : 15 and 1 : 75.

### Test example 13

After a single oral administration of the preparation samples obtained in Examples 24 and 48-52 to male Beagle dogs (3 for each preparation) 1 tablet of the samples for each dog, venous blood was taken at 0, 0.25, 0.5, 1, 2, 4, 6, 8, 10, 12 and 24 h, and the absolute bioavailability (F%) (relative to intravenous dose of compound A) was calculated from the area under the curve obtained from the points where the plasma concentration of compound A was taken as a function of time.

**Table 13 Effect of types of fatty acid salts on absorption of compound A**

| Formula | Cₘₐₓ (ng/ml) | F% |
|---|---|---|
| Example 1 | 2.60 ± 0.49 | 1.81 ± 0.01 |
| Example 24 | 15.9 ± 14 | 5.73 ± 3.3 |
| Example 48 | 1.80 ± 1.1 | 0.792 ± 0.31 |
| Example 49 | 13.7 ± 4.11 | 1.58 ± 1.11 |
| Example 50 | 3.54 ± 1.1 | 1.53 ± 0.31 |
| Example 51 | 0.837 ± 0.48 | 1.22 ± 0.38 |
| Example 52 | 1.48 ± 1.0 | 1.36 ± 0.22 |

### Results and discussion:

Fatty acid salts with different aliphatic chain lengths have different effects on bioavailability. Among the fatty acid salts with aliphatic chain lengths of C₆-C₁₆, only the fatty acid salts with aliphatic chain length of C₁₀ can significantly increase the bioavailability of compound A.

## Claims

1. An oral pharmaceutical composition, comprising:
a) compound A: 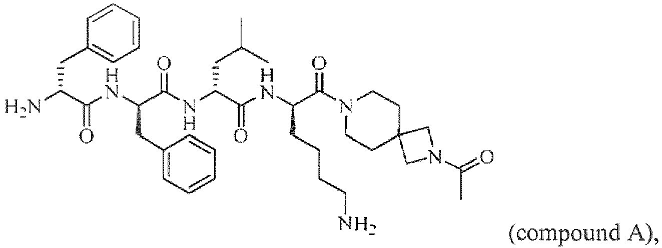 and
b) an absorption enhancer;
wherein the absorption enhancer is selected from one or more of N-[8-(2-hydroxybenzoyl)amino]octanoic acid or a pharmaceutically acceptable salt thereof, 4-[(4-chloro-2-hydroxy-benzoyl)amino]butyric acid or a pharmaceutically acceptable salt thereof, lauroyl-L-carnitine or a hydrochloride thereof, sodium caprylate, sodium caprate, capric acid, caprylocaproyl macrogolglyceride; preferably, the pharmaceutically acceptable salt is selected from sodium salt, potassium salt or calcium salt.

2. The oral pharmaceutical composition of claim 1, wherein the absorption enhancer is selected from N-[8-(2-hydroxybenzoyl)amino]octanoic acid or a pharmaceutically acceptable salt thereof;
preferably, the weight ratio of compound A to N-[8-(2-hydroxybenzoyl)amino]octanoic acid or a pharmaceutically acceptable salt thereof is 1 : 20-1 : 80, preferably 1 : 40-1 : 60, further preferably 1 : 40;
preferably, the oral pharmaceutical composition further comprises one or more non-active ingredients selected from
(1) a filler, which is preferably selected from one or more of microcrystalline cellulose, lactose, mannitol, anhydrous calcium hydrogenphosphate, pregelatinized starch, calcium hydrogenphosphate dihydrate;
(2) a binder, which is preferably selected from one or more of povidone, copovidone, a cellulose derivative, wherein the cellulose derivative is selected from one or more of hydroxypropylcellulose, hypromellose, methyl cellulose;
(3) a disintegrant, which is preferably selected from one or more of crospovidone, low substituted hydroxypropyl cellulose, croscarmellose sodium, sodium carboxymethyl starch;
(4) a pH regulator, which is preferably selected from one or more of citric acid, anhydrous citric acid, tartaric acid, fumaric acid, sodium citrate, calcium hydrogenphosphate, calcium carbonate;
(5) a surfactant, which is preferably selected from one or more of polyethylene glycol, poloxamer, phospholipid, Tween 80, Span 40, propylene glycol monolaurate, sodium dodecyl sulfate;
(6) a lubricant, which is preferably selected from one or more of magnesium stearate, stearic acid, sodium stearyl fumarate, glyceryl behenate.

3. The oral pharmaceutical composition of claim 1, wherein the absorption enhancer is selected from 4-[(4-chloro-2-hydroxy-benzoyl)amino]butyric acid or a pharmaceutically acceptable salt thereof;
preferably, the weight ratio of compound A to 4-[(4-chloro-2-hydroxybenzoyl)amino]butyric acid or a pharmaceutically acceptable salt thereof is 1 : 20-1 : 200, further preferably 1 : 40-1 : 80, more further preferably 1 : 40 or 3 : 200.

4. The oral pharmaceutical composition of claim 3, further comprising one or more non-active ingredients selected from
(1) a filler, which is preferably selected from one or more of microcrystalline cellulose, lactose, mannitol, anhydrous calcium hydrogenphosphate, pregelatinized starch, calcium hydrogenphosphate dihydrate;
(2) a binder, which is preferably selected from one or more of povidone, copovidone, a cellulose derivative, wherein the cellulose derivative is selected from one or more of hydroxypropylcellulose, hypromellose, methyl cellulose;
(3) a disintegrant, which is preferably selected from one or more of crospovidone, low substituted hydroxypropyl cellulose, croscarmellose sodium, sodium carboxymethyl starch;
(4) a pH regulator, which is preferably selected from one or more of citric acid, anhydrous citric acid, tartaric acid, fumaric acid, sodium citrate, calcium hydrogenphosphate, calcium carbonate;
(5) a surfactant, which is preferably selected from one or more of polyethylene glycol, poloxamer, phospholipid, Tween 80, Span 40, propylene glycol monolaurate, sodium dodecyl sulfate;
(6) a lubricant, which is preferably selected from one or more of magnesium stearate, stearic acid, sodium stearyl fumarate, glyceryl behenate.

5. The oral pharmaceutical composition of claim 4, wherein the non-active ingredient is a lubricant; preferably, the weight ratio of compound A: 4-[(4-chloro-2-hydroxybenzoyl)amino]butyric acid or a pharmaceutically acceptable salt thereof: the lubricant is 1 : (20-200) : (0.2-2), preferably 1 : 200 : 2;
preferably, the lubricant is magnesium stearate.

6. The oral pharmaceutical composition of claim 4, wherein the non-active ingredient is a filler and a binder; preferably, the weight ratio of compound A: 4-[(4-chloro-2-hydroxy-benzoyl)amino]butyric acid or a pharmaceutically acceptable salt thereof : the filler : the binder is 1 : (20-200) : 200 : 5, preferably 1 : 200 : 200 : 5;
preferably, the filler is microcrystalline cellulose, and the binder is povidone.

7. The oral pharmaceutical composition of claim 4, wherein the non-active ingredient is a filler, a binder and a lubricant; preferably, the weight ratio of compound A: 4-[(4-chloro-2-hydroxy-benzoyl)amino]butyric acid or a pharmaceutically acceptable salt thereof : the filler : the binder : the lubricant is 1 : (20-200) : 200 : 5 : (1-5), preferably 3 : 200 : 200 : 5 : 4,1 : 200 : 200 : 5 : 4 or 3 : 200 : 200 : 5 : 1;
preferably, the filler is selected from microcrystalline cellulose or anhydrous calcium hydrogenphosphate, the binder is povidone, and the lubricant is magnesium stearate.

8. The oral pharmaceutical composition of claim 4, wherein the non-active ingredient is a surfactant, a filler and a lubricant; preferably, the weight ratio of compound A: 4-[(4-chloro-2-hydroxy-benzoyl)amino]butyric acid or a pharmaceutically acceptable salt thereof : the surfactant : the filler : the lubricant is 3 : 200 : 6 : 200 : 1;
preferably, the surfactant is propylene glycol monolaurate and/or polyethylene glycol, the filler is anhydrous calcium hydrogenphosphate, and the lubricant is magnesium stearate.

9. The oral pharmaceutical composition of claim 1, wherein the absorption enhancer is selected from lauroyl-L-carnitine or a hydrochloride thereof;
preferably, the weight ratio of compound A to lauroyl-L-carnitine or a hydrochloride thereof is 1 : 10-1 : 150, preferably 1 : 10-1 : 100, further preferably 1 : 10-1 : 75;
preferably, the oral pharmaceutical compositionfurther comprises one or more non-active ingredients selected from
(1) a filler, which is preferably selected from one or more of microcrystalline cellulose, lactose, mannitol, anhydrous calcium hydrogenphosphate, pregelatinized starch, calcium hydrogenphosphate dihydrate;
(2) a binder, which is preferably selected from one or more of povidone, copovidone, a cellulose derivative, wherein the cellulose derivative is selected from one or more of hydroxypropylcellulose, hypromellose, methyl cellulose;
(3) a disintegrant, which is preferably selected from one or more of crospovidone, low substituted hydroxypropyl cellulose, croscarmellose sodium, sodium carboxymethyl starch;
(4) a pH regulator, which is preferably selected from one or more of citric acid, anhydrous citric acid, tartaric acid, fumaric acid, sodium citrate, calcium hydrogenphosphate, calcium carbonate;
(5) a surfactant, which is preferably selected from one or more of polyethylene glycol, poloxamer, phospholipid, Tween 80, Span 40, propylene glycol monolaurate, sodium dodecyl sulfate;
(6) a lubricant, which is preferably selected from one or more of magnesium stearate, stearic acid, sodium stearyl fumarate, glyceryl behenate;
preferably, the non-active ingredient is a pH regulator; preferably, the weight ratio of compound A : lauroyl-L-carnitine or a hydrochloride thereof : the pH regulator is 1 : (10-50) : 24;
preferably, the pH regulator is citric acid.

10. The oral pharmaceutical composition of claim 1, wherein the absorption enhancer is sodium caprate;
preferably, the weight ratio of compound A to sodium caprate is 1 : 50-1 : 200, preferably 1 : 100-1 : 200, further preferably 1 : 100;
preferably, the oral pharmaceutical composition further comprises one or more non-active ingredients selected from
(1) a filler, which is preferably selected from one or more of microcrystalline cellulose, lactose, mannitol, anhydrous calcium hydrogenphosphate, pregelatinized starch, calcium hydrogenphosphate dihydrate;
(2) a binder, which is preferably selected from one or more of povidone, copovidone, a cellulose derivative, wherein the cellulose derivative is selected from one or more of hydroxypropylcellulose, hypromellose, methyl cellulose;
(3) a disintegrant, which is preferably selected from one or more of crospovidone, low substituted hydroxypropyl cellulose, croscarmellose sodium, sodium carboxymethyl starch;
(4) a glyceride, which is preferably selected from one or more of monocaprylin, tricaprylin;
(5) a surfactant, which is preferably selected from one or more of polyethylene glycol, poloxamer, phospholipid, Tween 80, Span 40, propylene glycol monolaurate, sodium dodecyl sulfate;
(6) a lubricant, which is preferably selected from one or more of magnesium stearate, stearic acid, sodium stearyl fumarate, glyceryl behenate.

11. The oral pharmaceutical composition of claim 1, wherein the absorption enhancer is capric acid;
preferably, the oral pharmaceutical composition further comprises one or more non-active ingredients selected from
(1) a filler, which is preferably selected from one or more of microcrystalline cellulose, lactose, mannitol, anhydrous calcium hydrogenphosphate, pregelatinized starch, calcium hydrogenphosphate dihydrate;
(2) a binder, which is preferably selected from one or more of povidone, copovidone, a cellulose derivative, wherein the cellulose derivative is selected from one or more of hydroxypropylcellulose, hypromellose, methyl cellulose;
(3) a hydrophobic medium, which is preferably selected from one or more of coconut oil, castor oil, olive oil;
(4) a surfactant, which is preferably selected from one or more of caprylocaproyl macrogolglyceride, polyethylene glycol, poloxamer, phospholipid, Tween 80, Span 40, propylene glycol monolaurate, sodium dodecyl sulfate;
(5) a glyceride, which is preferably selected from one or more of monocaprylin, tricaprylin.

12. The oral pharmaceutical composition of claim 1, wherein the absorption enhancer is caprylocaproyl macrogolglyceride;
preferably, the weight ratio of compound A to caprylocaproyl macrogolglyceride is 1 : 600-1 : 3600, preferably 1 : 2400-1 : 3600, further preferably 1 : 2400.
preferably, the oral pharmaceutical composition further comprises one or more non-active ingredients selected from
(1) a hydrophobic medium, which is preferably selected from one or more of coconut oil, castor oil, olive oil;
(2) a surfactant, which is preferably selected from one or more of polyethylene glycol, poloxamer, phospholipid, Tween 80, Span 40, propylene glycol monolaurate, sodium dodecyl sulfate;
(3) a glyceride, which is preferably selected from one or more of monocaprylin, tricaprylin.

13. A method for preparing the oral pharmaceutical composition of any one of claims 1-12, comprising the following step: compound A, the absorption enhancer and other non-active ingredients are mixed directly, and then filled into capsules or compressed into tablets; or compound A, the absorption enhancer and other non-active ingredients are wet granulated and then filled into capsules or compressed into tablets; or compound A, the absorption enhancer and other hydrophilic non-active ingredients are wet granulated, then dispersed in a hydrophobic medium, and filled into capsules; or compound A, the absorption enhancer and other non-active ingredients are dissolved in purified water to prepare a solution; or compound A, the absorption enhancer and other non-active ingredients are dissolved, dried and pulverized, and then filled into capsules or compressed into tablets.

14. The oral pharmaceutical composition of any one of claims 1-12 for use in treating diseases or conditions related to the κ-opioid receptor;
preferably, the diseases or conditions related to the κ-opioid receptor are selected from pain, inflammation, itching, edema, hyponatremia, hypokalemia, intestinal obstruction, cough and glaucoma;
preferably, the pain is selected from neuropathic pain, somatic pain, visceral pain and skin pain;
preferably, the diseases or conditions are selected from arthritis pain, kidney stone pain, hysterotrismus, dysmenorrhea, endometriosis, dyspepsia, pain after surgery, pain after medical treatment, ocular pain, otitis pain, breakthrough cancer pain, and pain associated with GI disorders.

## Patentansprüche

1. Orale pharmazeutische Zusammensetzung, die Folgendes umfasst:
a) Verbindung A: 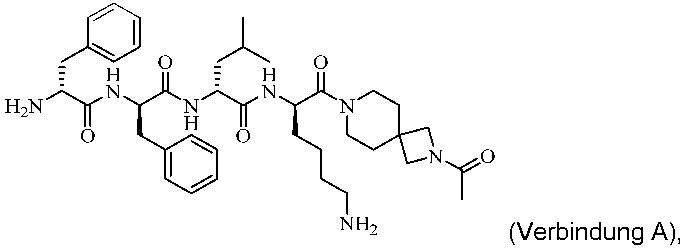 und
b) einen Absorptionsverstärker;
wobei der Absorptionsverstärker ausgewählt ist aus einem oder mehreren von N-[8-(2-Hydroxybenzoyl)amino]octansäure oder einem pharmazeutisch annehmbaren Salz davon, 4-[(4-Chlor-2-hydroxybenzoyl)amino]buttersäure oder einem pharmazeutisch annehmbaren Salz davon, Lauroyl-L-carnitin oder einem Hydrochlorid davon, Natriumcaprylat, Natriumcaprat, Caprinsäure, Caprylocaproyl-Macrogolglycerid; vorzugsweise ist das pharmazeutisch annehmbare Salz aus Natriumsalz, Kaliumsalz oder Kalziumsalz ausgewählt.

2. Orale pharmazeutische Zusammensetzung nach Anspruch 1, wobei der Absorptionsverstärker ausgewählt ist aus N-[8-(2-Hydroxybenzoyl)amino]octansäure oder einem pharmazeutisch annehmbaren Salz davon;
vorzugsweise beträgt das Gewichtsverhältnis von Verbindung A zu N-[8-(2-Hydroxybenzoyl)amino]octansäure oder einem pharmazeutisch annehmbaren Salz davon 1 : 20-1 : 80, vorzugsweise 1 : 40-1 : 60, weiter bevorzugt 1 : 40;
vorzugsweise umfasst die orale pharmazeutische Zusammensetzung ferner einen oder mehrere nicht-aktive Inhaltsstoffe, die ausgewählt sind aus
(1) einem Füllstoff, der vorzugsweise ausgewählt ist aus einem oder mehreren von mikrokristalliner Cellulose, Lactose, Mannitol, wasserfreiem Calciumhydrogenphosphat, vorgelatinierter Stärke, Calciumhydrogenphosphatdihydrat;
(2) einem Bindemittel, das vorzugsweise ausgewählt ist aus einem oder mehreren von Povidon, Copovidon, einem Cellulosederivat, wobei das Cellulosederivat aus einem oder mehreren von Hydroxypropylcellulose, Hypromellose, Methylcellulose ausgewählt ist;
(3) einem Sprengmittel, das vorzugsweise ausgewählt ist aus einem oder mehreren von Crospovidon, niedrig substituierter Hydroxypropylcellulose, Croscarmellosenatrium, Natriumcarboxymethylstärke;
(4) einem pH-Regulator, der vorzugsweise ausgewählt ist aus einem oder mehreren von Zitronensäure, wasserfreier Zitronensäure, Weinsäure, Fumarsäure, Natriumcitrat, Calciumhydrogenphosphat, Calciumcarbonat;
(5) einem Tensid, das vorzugsweise ausgewählt ist aus einem oder mehreren von Polyethylenglycol, Poloxamer, Phospholipid, Tween 80, Span 40, Propylenglycolmonolaurat, Natriumdodecylsulfat;
(6) einem Gleitmittel, das vorzugsweise ausgewählt ist aus einem oder mehreren von Magnesiumstearat, Stearinsäure, Natriumstearylfumarat, Glycerylbehenat.

3. Orale pharmazeutische Zusammensetzung nach Anspruch 1, wobei der Absorptionsverstärker ausgewählt ist aus 4-[(4-Chlor-2-hydroxybenzoyl)amino]buttersäure oder einem pharmazeutisch annehmbaren Salz davon;
vorzugsweise beträgt das Gewichtsverhältnis von Verbindung A zu 4-[(4-Chlor-2-hydroxybenzoyl)amino]buttersäure oder einem pharmazeutisch annehmbaren Salz davon 1 : 20-1 : 200, weiter vorzugsweise 1 : 40-1 : 80, noch weiter bevorzugt 1 : 40 oder 3 : 200.

4. Orale pharmazeutische Zusammensetzung nach Anspruch 3, ferner umfassend einen oder mehrere nicht-aktive Inhaltsstoffe, die ausgewählt sind aus
(1) einem Füllstoff, der vorzugsweise ausgewählt ist aus einem oder mehreren von mikrokristalliner Cellulose, Lactose, Mannitol, wasserfreiem Calciumhydrogenphosphat, vorgelatinierter Stärke, Calciumhydrogenphosphatdihydrat;
(2) einem Bindemittel, das vorzugsweise ausgewählt ist aus einem oder mehreren von Povidon, Copovidon, einem Cellulosederivat, wobei das Cellulosederivat aus einem oder mehreren von Hydroxypropylcellulose, Hypromellose, Methylcellulose ausgewählt ist;
(3) einem Sprengmittel, das vorzugsweise ausgewählt ist aus einem oder mehreren von Crospovidon, niedrig substituierter Hydroxypropylcellulose, Croscarmellosenatrium, Natriumcarboxymethylstärke;
(4) einem pH-Regulator, der vorzugsweise ausgewählt ist aus einem oder mehreren von Zitronensäure, wasserfreier Zitronensäure, Weinsäure, Fumarsäure, Natriumcitrat, Calciumhydrogenphosphat, Calciumcarbonat;
(5) einem Tensid, das vorzugsweise ausgewählt ist aus einem oder mehreren von Polyethylenglycol, Poloxamer, Phospholipid, Tween 80, Span 40, Propylenglycolmonolaurat, Natriumdodecylsulfat;
(6) einem Gleitmittel, das vorzugsweise ausgewählt ist aus einem oder mehreren von Magnesiumstearat, Stearinsäure, Natriumstearylfumarat, Glycerylbehenat.

5. Orale pharmazeutische Zusammensetzung nach Anspruch 4, wobei der nicht-aktive Inhaltsstoff ein Gleitmittel ist; vorzugsweise beträgt das Gewichtsverhältnis von Verbindung A : 4-[(4-Chlor-2-hydroxybenzoyl)amino]buttersäure oder einem pharmazeutisch annehmbaren Salz davon : dem Gleitmittel 1 : (20-200) : (0,2-2), vorzugsweise 1 : 200 : 2;
vorzugsweise handelt es sich bei dem Gleitmittel um Magnesiumstearat.

6. Orale pharmazeutische Zusammensetzung nach Anspruch 4, wobei der nicht-aktive Inhaltsstoff ein Füllstoff und ein Bindemittel ist; vorzugsweise beträgt das Gewichtsverhältnis von Verbindung A : 4-[(4-Chlor-2-hydroxybenzoyl)amino]buttersäure oder einem pharmazeutisch annehmbaren Salz davon : dem Füllstoff : dem Bindemittel 1 : (20-200) : 200 : 5, vorzugsweise 1 : 200 : 200 : 5;
vorzugsweise handelt es sich bei dem Füllstoff um mikrokristalline Cellulose, und bei dem Bindemittel handelt es sich um Povidon.

7. Orale pharmazeutische Zusammensetzung nach Anspruch 4, wobei der nicht-aktive Inhaltsstoff ein Füllstoff, ein Bindemittel und ein Gleitmittel ist; vorzugsweise beträgt das Gewichtsverhältnis von Verbindung A : 4-[(4-Chlor-2-hydroxybenzoyl)amino]buttersäure oder einem pharmazeutisch annehmbaren Salz davon : dem Füllstoff : dem Bindemittel : dem Gleitmittel 1 : (20-200) : 200 : 5 : (1-5), vorzugsweise 3 : 200 : 200 : 5 : 4, 1 : 200 : 200 : 5 : 4 oder 3 : 200 : 200 : 5 : 1;
vorzugsweise ist der Füllstoff ausgewählt aus mikrokristalliner Cellulose oder wasserfreiem Calciumhydrogenphosphat, bei dem Bindemittel handelt es sich um Povidon, und bei dem Gleitmittel handelt es sich um Magnesiumstearat.

8. Orale pharmazeutische Zusammensetzung nach Anspruch 4, wobei der nicht-aktive Inhaltsstoff ein Tensid, ein Füllstoff und ein Gleitmittel ist; vorzugsweise beträgt das Gewichtsverhältnis von Verbindung A : 4-[(4-Chlor-2-hydroxybenzoyl)amino]buttersäure oder einem pharmazeutisch annehmbaren Salz davon : dem Tensid : dem Füllstoff : dem Gleitmittel 3 : 200 : 6 : 200 : 1;
vorzugsweise handelt es sich bei dem Tensid um Propylenglycolmonolaurat und/oder Polyethylenglycol, bei dem Füllstoff handelt es sich um wasserfreies Calciumhydrogenphosphat, und bei dem Gleitmittel handelt es sich um Magnesiumstearat.

9. Orale pharmazeutische Zusammensetzung nach Anspruch 1, wobei der Absorptionsverstärker ausgewählt ist aus Lauroyl-L-carnitin oder einem Hydrochlorid davon;
vorzugsweise beträgt das Gewichtsverhältnis von Verbindung A zu Lauroyl-L-carnitin oder einem Hydrochlorid davon 1 : 10-1 : 150, vorzugsweise 1 : 10-1 : 100, weiter bevorzugt 1 : 10-1 : 75;
vorzugsweise umfasst die orale pharmazeutische Zusammensetzung ferner einen oder mehrere nicht-aktive Inhaltsstoffe, die ausgewählt sind aus
(1) einem Füllstoff, der vorzugsweise ausgewählt ist aus einem oder mehreren von mikrokristalliner Cellulose, Lactose, Mannitol, wasserfreiem Calciumhydrogenphosphat, vorgelatinierter Stärke, Calciumhydrogenphosphatdihydrat;
(2) einem Bindemittel, das vorzugsweise ausgewählt ist aus einem oder mehreren von Povidon, Copovidon, einem Cellulosederivat, wobei das Cellulosederivat aus einem oder mehreren von Hydroxypropylcellulose, Hypromellose, Methylcellulose ausgewählt ist;
(3) einem Sprengmittel, das vorzugsweise ausgewählt ist aus einem oder mehreren von Crospovidon, niedrig substituierter Hydroxypropylcellulose, Croscarmellosenatrium, Natriumcarboxymethylstärke;
(4) einem pH-Regulator, der vorzugsweise ausgewählt ist aus einem oder mehreren von Zitronensäure, wasserfreier Zitronensäure, Weinsäure, Fumarsäure, Natriumcitrat, Calciumhydrogenphosphat, Calciumcarbonat;
(5) einem Tensid, das vorzugsweise ausgewählt ist aus einem oder mehreren von Polyethylenglycol, Poloxamer, Phospholipid, Tween 80, Span 40, Propylenglycolmonolaurat, Natriumdodecylsulfat;
(6) einem Gleitmittel, das vorzugsweise ausgewählt ist aus einem oder mehreren von Magnesiumstearat, Stearinsäure, Natriumstearylfumarat, Glycerylbehenat;
vorzugsweise handelt es sich bei dem nicht-aktiven Inhaltsstoff um einen pH-Regulator; vorzugsweise beträgt das Gewichtsverhältnis von Verbindung A : Lauroyl-L-carnitin oder einem Hydrochlorid davon : dem pH-Regulator 1 : (10-50) : 24;
vorzugsweise handelt es sich bei dem pH-Regulator um Zitronensäure.

10. Orale pharmazeutische Zusammensetzung nach Anspruch 1, wobei es sich bei dem Absorptionsverstärker um Natriumcaprat handelt;
vorzugsweise beträgt das Gewichtsverhältnis von Verbindung A zu Natriumcaprat 1 : 50-1 : 200, vorzugsweise 1 : 100-1 : 200, weiter bevorzugt 1 : 100;
vorzugsweise umfasst die orale pharmazeutische Zusammensetzung ferner einen oder mehrere nicht-aktive Inhaltsstoffe, die ausgewählt sind aus
(1) einem Füllstoff, der vorzugsweise ausgewählt ist aus einem oder mehreren von mikrokristalliner Cellulose, Lactose, Mannitol, wasserfreiem Calciumhydrogenphosphat, vorgelatinierter Stärke, Calciumhydrogenphosphatdihydrat;
(2) einem Bindemittel, das vorzugsweise ausgewählt ist aus einem oder mehreren von Povidon, Copovidon, einem Cellulosederivat, wobei das Cellulosederivat aus einem oder mehreren von Hydroxypropylcellulose, Hypromellose, Methylcellulose ausgewählt ist;
(3) einem Sprengmittel, das vorzugsweise ausgewählt ist aus einem oder mehreren von Crospovidon, niedrig substituierter Hydroxypropylcellulose, Croscarmellosenatrium, Natriumcarboxymethylstärke;
(4) einem Glycerid, das vorzugsweise ausgewählt ist aus einem oder mehreren von Monocaprylin, Tricaprylin;
(5) einem Tensid, das vorzugsweise ausgewählt ist aus einem oder mehreren von Polyethylenglycol, Poloxamer, Phospholipid, Tween 80, Span 40, Propylenglycolmonolaurat, Natriumdodecylsulfat;
(6) einem Gleitmittel, das vorzugsweise ausgewählt ist aus einem oder mehreren von Magnesiumstearat, Stearinsäure, Natriumstearylfumarat, Glycerylbehenat.

11. Orale pharmazeutische Zusammensetzung nach Anspruch 1, wobei es sich bei dem Absorptionsverstärker um Caprinsäure handelt;
vorzugsweise umfasst die orale pharmazeutische Zusammensetzung ferner einen oder mehrere nicht-aktive Inhaltsstoffe, die ausgewählt sind aus
(1) einem Füllstoff, der vorzugsweise ausgewählt ist aus einem oder mehreren von mikrokristalliner Cellulose, Lactose, Mannitol, wasserfreiem Calciumhydrogenphosphat, vorgelatinierter Stärke, Calciumhydrogenphosphatdihydrat;
(2) einem Bindemittel, das vorzugsweise ausgewählt ist aus einem oder mehreren von Povidon, Copovidon, einem Cellulosederivat, wobei das Cellulosederivat aus einem oder mehreren von Hydroxypropylcellulose, Hypromellose, Methylcellulose ausgewählt ist;
(3) einem hydrophoben Medium, das vorzugsweise ausgewählt ist aus einem oder mehreren von Kokosöl, Rizinusöl, Olivenöl;
(4) einem Tensid, das vorzugsweise ausgewählt ist aus einem oder mehreren von Caprylocaproyl-Macrogolglycerid, Polyethylenglycol, Poloxamer, Phospholipid, Tween 80, Span 40, Propylenglycolmonolaurat, Natriumdodecylsulfat;
(5) einem Glycerid, das vorzugsweise ausgewählt ist aus einem oder mehreren von Monocaprylin, Tricaprylin.

12. Orale pharmazeutische Zusammensetzung nach Anspruch 1, wobei es sich bei dem Absorptionsverstärker um Caprylocaproyl-Macrogolglycerid handelt;
vorzugsweise beträgt das Gewichtsverhältnis von Verbindung A zu Caprylocaproyl-Macrogolclycerid 1 : 600-1 : 3600, vorzugsweise 1 : 2400-1 : 3600, weiter bevorzugt 1 : 2400.
vorzugsweise umfasst die orale pharmazeutische Zusammensetzung ferner einen oder mehrere nicht-aktive Inhaltsstoffe, die ausgewählt sind aus
(1) einem hydrophoben Medium, das vorzugsweise ausgewählt ist aus einem oder mehreren von Kokosöl, Rizinusöl, Olivenöl;
(2) einem Tensid, das vorzugsweise ausgewählt ist aus einem oder mehreren von Polyethylenglycol, Poloxamer, Phospholipid, Tween 80, Span 40, Propylenglycolmonolaurat, Natriumdodecylsulfat;
(3) einem Glycerid, das vorzugsweise ausgewählt ist aus einem oder mehreren von Monocaprylin, Tricaprylin.

13. Verfahren zur Herstellung der oralen pharmazeutischen Zusammensetzung nach einem der Ansprüche 1-12, mit dem folgenden Schritt: Verbindung A, der Absorptionsverstärker und andere nicht-aktive Inhaltsstoffe werden direkt vermischt und dann in Kapseln gefüllt oder zu Tabletten komprimiert; oder Verbindung A, der Absorptionsverstärker und andere nicht-aktive Inhaltsstoffe werden feuchtgranuliert und dann in Kapseln gefüllt oder zu Tabletten komprimiert; oder Verbindung A, der Absorptionsverstärker und andere hydrophile nicht-aktive Inhaltsstoffe werden feuchtgranuliert, dann in einem hydrophoben Medium dispergiert und in Kapseln gefüllt; oder Verbindung A, der Absorptionsverstärker und andere nicht-aktive Inhaltsstoffe werden in gereinigtem Wasser gelöst, um eine Lösung herzustellen; oder Verbindung A, der Absorptionsverstärker und andere nicht-aktive Inhaltsstoffe werden gelöst, getrocknet und pulverisiert und dann in Kapseln gefüllt oder zu Tabletten komprimiert.

14. Orale pharmazeutische Zusammensetzung nach einem der Ansprüche 1-12 zur Verwendung bei der Behandlung von Krankheiten oder Beschwerden in Verbindung mit dem κ-Opioidrezeptor;
vorzugsweise sind die Krankheiten oder Beschwerden in Verbindung mit dem κ-Opioidrezeptor ausgewählt aus Schmerzen, Entzündung, Juckreiz, Ödem, Hyponaträmie, Hypokalämie, Darmverschluss, Husten und Glaukom;
vorzugsweise sind die Schmerzen ausgewählt aus neuropathischen Schmerzen, somatischen Schmerzen, viszeralen Schmerzen und Hautschmerzen;
vorzugsweise sind die Krankheiten oder Beschwerden ausgewählt aus Arthritisschmerzen, Nierensteinschmerzen, Hysterotrismus, Dysmenorrhoe, Endometriose, Dyspepsie, Schmerzen nach einer Operation, Schmerzen nach einer medizinischen Behandlung, Augenschmerzen, Schmerzen bei Otitis, tumorbedingten Durchbruchschmerzen und Schmerzen in Verbindung mit gastrointestinalen Störungen.

## Revendications

1. Composition pharmaceutique orale, comprenant :
a) un composé A : 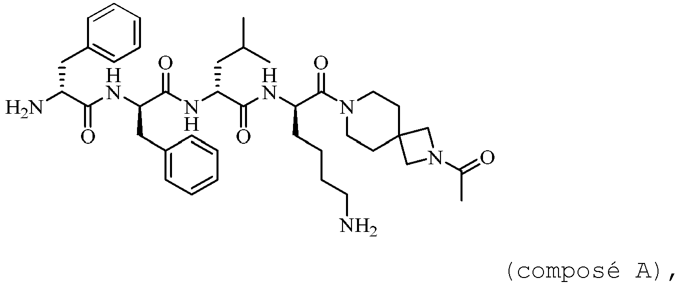 et
b) un promoteur d'absorption ;
dans laquelle le promoteur d'absorption est choisi parmi un ou plusieurs de l'acide N-[8-(2-hydroxybenzoyl)amino]octanoïque ou un sel pharmaceutiquement acceptable de celui-ci, de l'acide 4-[(4-chloro-2-hydroxy-benzoyl)amino]butyrique ou un sel pharmaceutiquement acceptable de celui-ci, de la lauroyl-L-carnitine ou un hydrochlorure de celle-ci, du caprylate de sodium, du caprate de sodium, de l'acide caprique, du caprylocaproyl macrogolglycéride ; de préférence, le sel pharmaceutiquement acceptable est choisi parmi le sel de sodium, le sel de potassium ou le sel de calcium.

2. Composition pharmaceutique orale selon la revendication 1, dans laquelle le promoteur d'absorption est choisi parmi l'acide N-[8-(2-hydroxybenzoyl)amino]octanoïque ou un sel pharmaceutiquement acceptable de celui-ci ;
de préférence, le rapport pondéral du composé A sur l'acide N-[8-(2-hydroxybenzoyl)amino]octanoïque ou un sel pharmaceutiquement acceptable de celui-ci est 1 : 20-1 : 80, de préférence 1 : 40-1 : 60, de préférence encore 1 : 40 ;
de préférence, la composition pharmaceutique orale comprend en outre un ou plusieurs ingrédients non actifs choisis parmi
(1) une charge, qui est de préférence choisie parmi un ou plusieurs de la cellulose microcristalline, du lactose, du mannitol, de l'hydrogénophosphate de calcium anhydre, de l'amidon prégélatinisé, de l'hydrogénophosphate de calcium dihydraté ;
(2) un liant, qui est de préférence choisi parmi un ou plusieurs de la povidone, de la copovidone, d'un dérivé de cellulose, dans laquelle le dérivé de cellulose est choisi parmi un ou plusieurs de l'hydroxypropylcellulose, de l'hypromellose, de la méthylcellulose ;
(3) un désintégrant, qui est de préférence choisi parmi un ou plusieurs de la crospovidone, de l'hydroxypropyl cellulose faiblement substituée, du croscarmellose sodique, du carboxyméthylamidon sodique ;
(4) un régulateur de pH, qui est de préférence choisi parmi un ou plusieurs de l'acide citrique, de l'acide citrique anhydre, de l'acide tartrique, de l'acide fumarique, du citrate de sodium, de l'hydrogénophosphate de calcium, du carbonate de calcium ;
(5) un tensioactif, qui est de préférence choisi parmi un ou plusieurs du polyéthylène glycol, du poloxamère, du phospholipide, du Tween 80, du Span 40, du monolaurate de propylène glycol, du laurylsulfate de sodium ;
(6) un lubrifiant, qui est de préférence choisi parmi un ou plusieurs du stéarate de magnésium, de l'acide stéarique, du stéaryl fumarate de sodium, du glycéryl béhénate.

3. Composition pharmaceutique orale selon la revendication 1, dans laquelle le promoteur d'absorption est choisi parmi l'acide 4-[(4-chloro-2-hydroxy-benzoyl)amino]butyrique ou un sel pharmaceutiquement acceptable de celui-ci ;
de préférence, le rapport pondéral du composé A sur l'acide 4-[(4-chloro-2-hydroxy-benzoyl)amino]butyrique ou un sel pharmaceutiquement acceptable de celui-ci est 1 : 20-1 : 200, de préférence encore 1 : 40-1 : 80, plus préférentiellement 1 : 40 ou 3 : 200.

4. Composition pharmaceutique orale selon la revendication 3, comprenant en outre un ou plusieurs ingrédients non actifs choisis parmi
(1) une charge, qui est de préférence choisie parmi un ou plusieurs de la cellulose microcristalline, du lactose, du mannitol, de l'hydrogénophosphate de calcium anhydre, de l'amidon prégélatinisé, de l'hydrogénophosphate de calcium dihydraté ;
(2) un liant, qui est de préférence choisi parmi un ou plusieurs de la povidone, de la copovidone, d'un dérivé de cellulose, dans laquelle le dérivé de cellulose est choisi parmi un ou plusieurs de l'hydroxypropylcellulose, de l'hypromellose, de la méthylcellulose ;
(3) un désintégrant, qui est de préférence choisi parmi un ou plusieurs de la crospovidone, de l'hydroxypropyl cellulose faiblement substituée, du croscarmellose sodique, du carboxyméthylamidon sodique ;
(4) un régulateur de pH, qui est de préférence choisi parmi un ou plusieurs de l'acide citrique, de l'acide citrique anhydre, de l'acide tartrique, de l'acide fumarique, du citrate de sodium, de l'hydrogénophosphate de calcium, du carbonate de calcium ;
(5) un tensioactif, qui est de préférence choisi parmi un ou plusieurs du polyéthylène glycol, du poloxamère, du phospholipide, du Tween 80, du Span 40, du monolaurate de propylène glycol, du laurylsulfate de sodium ;
(6) un lubrifiant, qui est de préférence choisi parmi un ou plusieurs du stéarate de magnésium, de l'acide stéarique, du stéaryl fumarate de sodium, du glycéryl béhénate.

5. Composition pharmaceutique orale selon la revendication 4, dans laquelle l'ingrédient non actif est un lubrifiant ; de préférence, le rapport pondéral du composé A : acide 4-[(4-chloro-2-hydroxy-benzoyl)amino]butyrique ou un sel pharmaceutiquement acceptable de celui-ci : le lubrifiant est 1 : (20-200) : (0,2-2) de préférence 1 : 200 : 2 ;
de préférence, le lubrifiant est du stéarate de magnésium.

6. Composition pharmaceutique orale selon la revendication 4, dans laquelle l'ingrédient non actif est une charge et un liant ; de préférence, le rapport pondéral du composé A : acide 4-[(4-chloro-2-hydroxy-benzoyl)amino]butyrique ou un sel pharmaceutiquement acceptable de celui-ci : la charge : le liant est 1 : (20-200) : 200 : 5, de préférence 1 : 200 : 200 : 5 ;
de préférence, la charge est de la cellulose microcristalline, et le liant est de la povidone.

7. Composition pharmaceutique orale selon la revendication 4, dans laquelle l'ingrédient non actif est une charge, un liant et un lubrifiant ; de préférence, le rapport pondéral du composé A : acide 4-[(4-chloro-2-hydroxy-benzoyl)amino]butyrique ou un sel pharmaceutiquement acceptable de celui-ci : la charge : le liant : le lubrifiant est 1 : (20-200) : 200 : 5 : (1-5), de préférence 3 : 200 200 : 5 : 4, 1 : 200 : 200 : 5 : 4 ou 3 : 200 : 200 : 5 : 1 ;
de préférence, la charge est choisie parmi la cellulose microcristalline ou l'hydrogénophosphate de calcium anhydre, le liant est la povidone et le lubrifiant est le stéarate de magnésium.

8. Composition pharmaceutique orale selon la revendication 4, dans laquelle l'ingrédient non actif est un tensioactif, une charge et un lubrifiant ; de préférence, le rapport pondéral du composé A : acide 4-[(4-chloro-2-hydroxy-benzoyl)amino]butyrique ou un sel pharmaceutiquement acceptable de celui-ci : le tensioactif : la charge : le lubrifiant est 3 : 200 : 6 : 200 : 1 ;
de préférence, le tensioactif est du monolaurate de propylène glycol et/ou du polyéthylène glycol, la charge est de l'hydrogénophosphate de calcium anhydre, et le lubrifiant est du stéarate de magnésium.

9. Composition pharmaceutique orale selon la revendication 1, dans laquelle le promoteur d'absorption est choisi parmi la lauroyl-L-carnitine ou un hydrochlorure de celle-ci ;
de préférence, le rapport pondéral du composé A sur la lauroyl-L-carnitine ou un hydrochlorure de celle-ci est 1 : 10-1 : 150, de préférence 1 : 10-1 : 100, de préférence encore 1 : 10-1 : 75 ;
de préférence, la composition pharmaceutique orale comprend en outre un ou plusieurs ingrédients non actifs choisis parmi
(1) une charge, qui est de préférence choisie parmi un ou plusieurs de la cellulose microcristalline, du lactose, du mannitol, du phosphate d'hydrogène calcique anhydre, de l'amidon prégélatinisé, du phosphate d'hydrogène calcique dihydraté ;
(2) un liant, qui est de préférence choisi parmi un ou plusieurs de la povidone, de la copovidone, d'un dérivé de cellulose, dans laquelle le dérivé de cellulose est choisi parmi un ou plusieurs de l'hydroxypropylcellulose, de l'hypromellose, de la méthylcellulose ;
(3) un désintégrant, qui est de préférence choisi parmi un ou plusieurs de la crospovidone, de l'hydroxypropyl cellulose faiblement substituée, du croscarmellose sodique, du carboxyméthylamidon sodique ;
(4) un régulateur de pH, qui est de préférence choisi parmi un ou plusieurs de l'acide citrique, de l'acide citrique anhydre, de l'acide tartrique, de l'acide fumarique, du citrate de sodium, de l'hydrogénophosphate de calcium, du carbonate de calcium ;
(5) un tensioactif, qui est de préférence choisi parmi un ou plusieurs du polyéthylène glycol, du poloxamère, du phospholipide, du Tween 80, du Span 40, du monolaurate de propylène glycol, du laurylsulfate de sodium ;
(6) un lubrifiant, qui est de préférence choisi parmi un ou plusieurs du stéarate de magnésium, de l'acide stéarique, du stéaryl fumarate de sodium, du glycéryl béhénate ;
de préférence, l'ingrédient non actif est un régulateur de pH ; de préférence, le rapport pondéral du composé A : lauroyl-L-carnitine ou un hydrochlorure de celle-ci : le régulateur de pH est 1 : (10-50) : 24 ;
de préférence, le régulateur de pH est de l'acide citrique.

10. Composition pharmaceutique orale selon la revendication 1, dans laquelle le promoteur d'absorption est le caprate de sodium ;
de préférence, le rapport pondéral du composé A sur le caprate de sodium est 1 : 50-1: 200, de préférence 1 : 100-1 : 200, de préférence encore 1 : 100 ;
de préférence, la composition pharmaceutique orale comprend en outre un ou plusieurs ingrédients non actifs choisis parmi
(1) une charge, qui est de préférence choisie parmi un ou plusieurs de la cellulose microcristalline, du lactose, du mannitol, de l'hydrogénophosphate de calcium anhydre, de l'amidon prégélatinisé, de l'hydrogénophosphate de calcium dihydraté ;
(2) un liant, qui est de préférence choisi parmi un ou plusieurs de la povidone, de la copovidone, d'un dérivé de cellulose, dans laquelle le dérivé de cellulose est choisi parmi un ou plusieurs de l'hydroxypropylcellulose, de l'hypromellose, de la méthylcellulose ;
(3) un désintégrant, qui est de préférence choisi parmi un ou plusieurs de la crospovidone, de l'hydroxypropyl cellulose faiblement substituée, du croscarmellose sodique, du carboxyméthylamidon sodique ;
(4) un glycéride, qui est de préférence choisi parmi une ou plusieurs de la monocapryline, de la tricapryline ;
(5) un tensioactif, qui est de préférence choisi parmi un ou plusieurs du polyéthylène glycol, du poloxamère, du phospholipide, du Tween 80, du Span 40, du monolaurate de propylène glycol, du laurylsulfate de sodium ;
(6) un lubrifiant, qui est de préférence choisi parmi un ou plusieurs du stéarate de magnésium, de l'acide stéarique, du stéaryl fumarate de sodium, du glycéryl béhénate.

11. Composition pharmaceutique orale selon la revendication 1, dans laquelle le promoteur d'absorption est l'acide caprique ;
de préférence, la composition pharmaceutique orale comprend en outre un ou plusieurs ingrédients non actifs choisis parmi
(1) une charge, qui est de préférence choisie parmi un ou plusieurs de la cellulose microcristalline, du lactose, du mannitol, de l'hydrogénophosphate de calcium anhydre, de l'amidon prégélatinisé, de l'hydrogénophosphate de calcium dihydraté ;
(2) un liant, qui est de préférence choisi parmi un ou plusieurs de la povidone, de la copovidone, d'un dérivé de cellulose, dans laquelle le dérivé de cellulose est choisi parmi un ou plusieurs de l'hydroxypropylcellulose, de l'hypromellose, de la méthylcellulose ;
(3) un milieu hydrophobe, qui est de préférence choisi parmi une ou plusieurs de l'huile de noix de coco, de l'huile de ricin, de l'huile d'olive ;
(4) un tensioactif, qui est de préférence choisi parmi un ou plusieurs du caprylocaproyl macrogolglycéride, du polyéthylène glycol, du poloxamère, du phospholipide, du Tween 80, du Span 40, du monolaurate de propylène glycol, du laurylsulfate de sodium ;
(5) un glycéride, qui est de préférence choisi parmi une ou plusieurs de la monocapryline, de la tricapryline.

12. Composition pharmaceutique orale selon la revendication 1, dans laquelle le promoteur d'absorption est le caprylocaproyl macrogolglycéride ;
de préférence, le rapport pondéral du composé A sur le caprylocaproyl macrogolglycéride est 1 : 600-1 : 3600, de préférence 1: 2400-1 : 3600, de préférence encore 1 : 2400.
de préférence, la composition pharmaceutique orale comprend en outre un ou plusieurs ingrédients non actifs choisis parmi
(1) un milieu hydrophobe, qui est de préférence choisi parmi une ou plusieurs de l'huile de noix de coco, de l'huile de ricin, de l'huile d'olive ;
(2) un tensioactif, qui est de préférence choisi parmi un ou plusieurs du polyéthylène glycol, du poloxamère, du phospholipide, du Tween 80, du Span 40, du monolaurate de propylène glycol, du laurysulfate de sodium ;
(3) un glycéride, qui est de préférence choisi parmi une ou plusieurs de la monocapryline, de la tricapryline.

13. Procédé de préparation de la composition pharmaceutique orale selon l'une quelconque des revendications 1 à 12, comprenant l'étape suivante : le composé A, le promoteur d'absorption et les autres ingrédients non actifs sont mélangés directement, puis versés dans des gélules ou compressés en comprimés ; ou le composé A, le promoteur d'absorption et les autres ingrédients non actifs sont granulés par voie humide puis versés dans des gélules ou compressés en comprimés ; ou le composé A, le promoteur d'absorption et d'autres ingrédients non actifs hydrophiles sont granulés par voie humide, puis dispersés dans un milieu hydrophobe, et versés dans des gélules ; ou le composé A, le promoteur d'absorption et d'autres ingrédients non actifs sont dissous dans de l'eau purifiée pour préparer une solution ; ou le composé A, le promoteur d'absorption et d'autres ingrédients non actifs sont dissous, séchés et pulvérisés, puis versés dans des gélules ou compressés en comprimés.

14. Composition pharmaceutique orale selon l'une quelconque des revendications 1 à 12 pour une utilisation dans le traitement de maladies ou d'affections liées au récepteur opioïde kappa ;
de préférence, les maladies ou affections liées au récepteur opioïde kappa sont choisies parmi la douleur, l'inflammation, les démangeaisons, l'œdème, l'hyponatrémie, l'hypokaliémie, l'obstruction intestinale, la toux et le glaucome ;
de préférence, la douleur est choisie parmi la douleur neuropathique, la douleur somatique, la douleur viscérale et la douleur cutanée ;
de préférence, les maladies ou affections sont choisies parmi la douleur liée à l'arthrite, la douleur liée aux calculs rénaux, l'hystérotrisme, la dysménorrhée, l'endométriose, la dyspepsie, la douleur postopératoire, la douleur après un traitement médical, la douleur oculaire, la douleur liée à l'otite, la douleur liée à l'apparition d'un cancer, et la douleur associée aux troubles gastro-intestinaux.
